# EUROPEAN PATENT SPECIFICATION

(11) **EP 3 946 299 B1**
(45) Date of publication and mention of the grant of the patent: **11.11.2026**
(21) Application number: 20782643.9
(22) Date of filing: 27.03.2020
(51) Int. Cl.: C07C 233/42, C07C 235/38, C07C 259/06, C07C 311/20, C07D 209/14, C07D 233/74

(54) **PERIPHERAL ALKYL AND ALKENYL CHAINS EXTENDED BENZENE DERIVATIVES AND PHARMACEUTICAL COMPOSITION INCLUDING THE SAME**
MIT PERIPHEREN ALKYL- UND ALKENYLKETTEN VERLÄNGERTE BENZOLDERIVATE UND PHARMAZEUTISCHE ZUSAMMENSETZUNGEN DAMIT
DÉRIVÉS BENZÉNIQUES À CHAÎNES ALKYLE ET ALCÉNYLE PÉRIPHÉRIQUES ÉTENDUES ET COMPOSITION PHARMACEUTIQUE COMPRENANT CEUX-CI

(30) Priority: 29.03.2019 US 201962825977 P
(43) Date of publication of application: 09.02.2022
(73) Proprietor: Taiwanj Pharmaceuticals Co., Ltd., Zhubei City, Hsinchu County 30261 (TW)
(72) Inventor: JIANG, Yan-feng, Kaohsiung, 832 (TW); LIU, Meng-hsien, Toufen Miaoli County, 351 (TW); CHANG, Chia-hao, New Taipei, 23562 (TW); LIU, Hao, Shiuan, Taichung, 429 (TW); SHIH, Ying-chu, Zhubei Hsinchu County, 30261 (TW); LIU, Sheng, Hung, Zhubei Hsinchu County, 30261 (TW); WANG, Chiung, Wen, Zhubei Hsinchu County, 30261 (TW); HUANG, Ting-ni, Taipei, 11647 (TW); YANG, Syaulan, S., Zhubei Hsinchu County, 30261 (TW)
(74) Representative: Becker Kurig & Partner Patentanwälte mbB
(86) International application number: PCT/US2020/025099
(87) International publication number: WO 2020/205455

(56) References cited:
- WO-A1-2019/051222
- WO-A1-2019/051222
- WO-A1-97/20806
- CN-A- 101 979 090
- US-A1- 2002 065 282
- US-A1- 2018 016 274
- US-B2- 7 763 662
- US-B2- 8 580 822
- US-B2- 9 676 691
- KOCH K ET AL: "HYDROXY-DIRECTED HYDROALUMINATIONS: A STEREOSELECTIVE APPROACH TO CYCLOALKANOLS FROM BETA-ARYL ENONES", TETRAHEDRON LETTERS, ELSEVIER, AMSTERDAM , NL, vol. 35, no. 8, 21 February 1994 (1994-02-21), pages 1137 - 1140, XP000673082, ISSN: 0040-4039, DOI: 10.1016/0040-4039(94)88006-9
- YEE S W ET AL: "Synthesis and CYP24 inhibitory activity of 2-substituted-3,4-dihydro-2H-naphthalen-1-one (tetralone) derivatives", BIOORGANIC & MEDICINAL CHEMISTRY LETTERS, ELSEVIER, AMSTERDAM, NL, vol. 14, no. 22, 15 November 2004 (2004-11-15), pages 5651 - 5654, XP004598611, ISSN: 0960-894X, DOI: 10.1016/J.BMCL.2004.08.040
- SOOK WAH YEE ET AL: "Novel Tetralone-Derived Retinoic Acid Metabolism Blocking Agents: Synthesis and in Vitro Evaluation with Liver Microsomal and MCF-7 CYP26A1 Cell Assays", JOURNAL OF MEDICINAL CHEMISTRY, vol. 48, no. 23, 1 November 2005 (2005-11-01), pages 7123 - 7131, XP55015742, ISSN: 0022-2623, DOI: 10.1021/jm0501681
- HUANG ZHIYAN ET AL: "Arene CH?O Hydrogen Bonding: A Stereocontrolling Tool in Palladium-Catalyzed Arylation and Vinylation of Ketones", ANGEWANDTE CHEMIE INTERNATIONAL EDITION, vol. 52, no. 18, 3 April 2013 (2013-04-03), pages 4906 - 4911, XP55943100, ISSN: 1433-7851, DOI: 10.1002/anie.201300621
- ANONYMOUS: "6-Methoxy-2-[[4-(trifluoromethyl)phenyl]methylidene]-3,4-dihydronaphthalen-1-one | C19H15F3O2 - PubChem", PUBCHEM CID : 2732770, 19 July 2005 (2005-07-19), pages 1 - 8, XP55746780, Retrieved from the Internet <URL:https://pubchem.ncbi.nlm.nih.gov/compound/2732770> [retrieved on 20201104]
- DATABASE PUBCHEM 16 September 2004 (2004-09-16), XP055746778, Database accession no. 22819
- DATABASE PUBCHEM 19 July 2005 (2005-07-19), XP055746780, Database accession no. 2732770

## Description

### BACKGROUND

### Technical Field

The present disclosure relates to peripheral alkyl and alkenyl chains extended benzene derivatives and pharmaceutical compositions including the same, in particular to a compound of Formula (I-1) as defined in the claims. The peripheral alkyl and alkenyl chains extended benzene derivatives are useful as a dual autotaxin (ATX) / histone deacetylase (HDAC) inhibitor.

### Description of Related Art

Histones form the core of nucleosomes, the DNA/protein complexes that are the subunits of eukaryotic chromatin. Histones' N-terminal "tails" are subject to a variety of posttranslational modifications, including phosphorylation, methylation, ubiquitination, ADP-ribosylation and acetylation. Histone deacetylases (HDACs) are responsible for removal of these acetyl groups, and play an important role in the regulation of gene expression. Human HDAC1 was the first protein to be linked to histone deacetylase activity. Aberrant expression of HDACs is observed in various human cancers. HDAC inhibitors could become useful as a therapeutic or prophylactic agent for diseases caused by abnormal gene expression such as inflammatory disorders or cancers.

Autotaxin (ATX) is a secreted enzyme that in humans is encoded by the ENPP2 gene, and is known as ectonucleotide pyrophosphatase/phosphodiesterase family member 2 (NPP2 or ENPP2) or lysophospholipase D. Autotaxin has lysophospholipase D activity that converts lysophosphatidylcholine into lysophosphatidic acid (LPA). Autotaxin is a ~120 kDa secreted enzyme that is important in generating the lipid signaling molecule LPA. The ATX-LPA axis is involved in a range of pathophysiological functions, including, for example, embryonic development, chronic inflammatory disease, or cancer biology. In cancer biology, LPA signaling is being recognized as a central mediator of the progression of chronic inflammation in the establishment of a tumor microenvironment which promotes cancer growth. So far, there is no approved cancer therapy that target the ATX-LPA axis.

Previous studies have shown that HDAC inhibitors may potentially induce autotaxin expression. However, dual inhibitors of HDAC and autotaxin have rarely been reported. Therefore, development of dual inhibitors of HDAC and autotaxin is desired, as they have great potential in the prophylaxis and treatment of various diseases, disorders etc.
WO 2019/051222 A1 discloses benzene fused heterocyclic derivatives as autotaxin inhibitors, and pharmaceutical compositions including the same.

### SUMMARY

In some embodiments of the present disclosure, a compound, which is a peripheral alkyl and alkenyl chains extended benzene derivative is described. The compound is represented by Formula (I-1), or its geometric isomers, enantiomers, diastereomers, racemates, pharmaceutically acceptable salts, or solvates thereof:

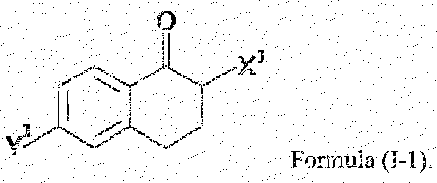

In Formula (I-1), X¹ is hydrogen or R¹-A¹ , wherein R¹ is a single bond or a double bond, A¹ is an aryl optionally substituted with -CF₃; Y¹ is a -NHC(O)R²C(O)NHR³, -R²C(O)NHR³, -R²C(O)NHR⁴C(O)NHR³, -C(O)NHR²C(O)NHR³, -OCH₂R⁵C(O)NHR³, -OCH₂R⁵R²C(O)NHR³, or -C(O)R⁶C(O)R²C(O)NHR³, , wherein R³ is independently selected from a group consisting of OH, an aryl optionally substituted with at least one of a halogen, an alkyl, an alkoxy or -NH₂, and when R³ is an aryl substituted with -NH₂, any hydrogen in the NH₂ may be optionally substituted with alkyl, R² and R⁴ are independently selected from a group consisting of C₁-C₁₀ alkyl, C₁-C₁₀ alkenyl, and C₁-C₁₀ alkoxy, R⁵ is an aryl and R⁶ is a heterocyclic alkyl. When Y¹ is OCH₂R⁵C(O)NHR³, R³ is an aryl optionally substituted with at least one of a halogen, an alkyl, an alkoxy or -NH₂.

The present disclosure further provides a pharmaceutical composition that includes a therapeutically effective amount of the compound of Formula (I-1) and a pharmaceutically acceptable carrier.

The compounds provided in the present disclosure may be used as dual HDAC / autotaxin inhibitors.

To make the aforementioned more comprehensible, several embodiments accompanied with drawings are described in detail as follows.

### DESCRIPTION OF THE EMBODIMENTS

The compounds of the present disclosure are described in detail in the following embodiments.

In the present disclosure, when a range of values is listed, it is intended to encompass each value and sub-range within the range. For example, "C₁-C₄" is intended to encompass, C₁, C₂, C₃, C₄, C₁-C₄, C₁-C₃, C₁-C₂, C₂-C₄, C₂-C₃, and C₃-C₄.

Unless otherwise indicated, the term "alkyl" means a straight chain and/or branched hydrocarbon having from 1 to 20 (e.g., 1 to 10, 1 to 9, 1 to 8, 1 to 7, 1 to 6, 1 to 5, 1 to 4, 1 to 3, 1 to 2, or 1) carbon atoms. Alkyl moieties having from 1 to 4 carbons (C₁-C₄ alkyl) are referred to as "lower alkyl." Examples of alkyl groups include methyl, ethyl, propyl, isopropyl, n-butyl, t-butyl, isobutyl, 2-isopropyl-3-methyl butyl, pentyl, pentan-2-yl, hexyl, isohexyl, heptyl, heptan-2-yl, 4,4-dimethylpentyl, octyl, 2,2,4-trimethylpentyl, nonyl, decyl, undecyl and dodecyl.

"Heterocyclic alkyl" refers to a radical of a 3- to 10- membered non-aromatic ring system having ring carbon atoms and 1 to 4 ring heteroatoms, wherein each heteroatom is independently selected from nitrogen, oxygen, sulfur, phosphorus, and silicon ("3-10 membered heterocyclic alkyl"). In heterocyclyl groups that contain one or more nitrogen atoms, the point of attachment can be a carbon or nitrogen atom, as valency permits. In some embodiments, a heterocyclyl group is a 5-8 membered non-aromatic ring system having ring carbon atoms and 1-4 ring heteroatoms, wherein each heteroatom is independently selected from nitrogen, oxygen, and sulfur. In some embodiments, a heterocyclic alkyl group is a 5-6 membered non-aromatic ring system having ring carbon atoms and 1-4 ring heteroatoms, wherein each heteroatom is independently selected from nitrogen, oxygen, and sulfur. In some embodiments, the 5-6 membered heterocyclyl has 1-3 ring heteroatoms selected from nitrogen, oxygen, and sulfur. In some embodiments, the 5-6 membered heterocyclyl has 1-2 ring heteroatoms selected from nitrogen, oxygen, and sulfur. In some embodiments, the 5-6 membered heterocyclic alkyl has one ring heteroatom selected from nitrogen, oxygen, and sulfur. Exemplary 5-membered heterocyclyl groups containing one heteroatom include tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl groups containing two heteroatoms include dioxolanyl, oxasulfuranyl, disulfuranyl, and oxazolidin-2-one. Exemplary 5- membered heterocyclyl groups containing three heteroatoms include triazolinyl, oxadiazolinyl, and thiadiazolinyl. Exemplary 6-membered heterocyclyl groups containing one heteroatom include piperidinyl, tetrahydropyranyl, dihydropyridinyl, and thianyl. Exemplary 6-membered heterocyclyl groups containing two heteroatoms include piperazinyl, morpholinyl, dithianyl, dioxanyl. Exemplary 6-membered heterocyclyl groups containing two heteroatoms include triazinanyl. Exemplary 7-membered heterocyclyl groups containing one heteroatom include azepanyl, oxepanyl and thiepanyl. Exemplary 8- membered heterocyclyl groups containing one heteroatom include azocanyl, oxecanyl and thiocanyl.

Unless otherwise indicated, the term "aryl" means an aromatic ring or a partially aromatic ring system composed of carbon and hydrogen atoms. An aryl moiety may comprise multiple rings bound or fused together. Examples of aryl moieties include naphthyl, and phenyl.

Unless otherwise indicated, the term "alkoxy" or "alkoxyl" means an -O-alkyl group. Examples of alkoxy groups include -OCH₃, -OCH₂CH₃, -O(CH₂)₂CH₃, -O(CH₂)₃CH₃, -O(CH₂)₄CH₃, and -O(CH₂)₅CH₃. The term "lower alkoxy" refers to -O-(lower alkyl), such as -OCH₃ and -OCH₂CH₃.

Unless otherwise indicated, the terms "halogen" and "halo" encompass fluoro-, chloro-, bromo-, and iodo- groups.

Unless otherwise indicated, the term "substituted," when used to describe a chemical structure or moiety, refers to a derivative of that structure or moiety wherein one or more of its hydrogen atoms is substituted with an atom, chemical moiety or functional group.

The term "solvate" refers to forms of the compound that are associated with a solvent, usually by a solvolysis reaction. This physical association may include hydrogen bonding. Conventional solvents include water, methanol, ethanol, acetic acid, dimethyl sulfoxide (DMSO), tetrahydrofuran (THF), and diethyl ether, . The compounds described herein may be prepared, e.g., in crystalline form, and may be solvated. Suitable solvates include pharmaceutically acceptable solvates and further include both stoichiometric solvates and non-stoichiometric solvates. In certain instances, the solvate will be capable of isolation, for example, when one or more solvent molecules are incorporated in the crystal lattice of a crystalline solid. "Solvate" encompasses both solution-phase and isolatable solvates. Representative solvates include hydrates, ethanolates, and methanolates.

The term "hydrate" refers to a compound which is associated with water. Typically, the number of the water molecules contained in a hydrate of a compound is in a definite ratio to the number of the compound molecules in the hydrate. Therefore, a hydrate of a compound may be represented, for example, by the general formula R·x H₂O, wherein R is the compound, and x is a number greater than 0. A given compound may form more than one type of hydrate, including, e.g., monohydrates (x is 1), lower hydrates (x is a number greater than 0 and smaller than 1, e.g., hemihydrates (R·0.5 H₂O)), and polyhydrates (x is a number greater than 1, e.g., dihydrates (R·2 H₂O) and hexahydrates (R·6 H₂O)).

It is also to be understood that compounds that have the same molecular formula but differ in the nature or sequence of bonding of their atoms or the arrangement of their atoms in space are termed "constitutional isomers" (not according to the invention). Isomers that differ in the arrangement of their atoms in space are termed "stereoisomers".

Stereoisomers that are not mirror images of one another are termed "diastereomers" and those that are non-superimposable mirror images of each other are termed "enantiomers". When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (i.e., as (+) or (-)-isomers respectively). A chiral compound can exist as either individual enantiomer or as a mixture thereof. A mixture containing equal proportions of the enantiomers is called a "racemic mixture".

It should also be noted that if the stereochemistry of a structure or a portion of a structure is not indicated with, for example, bold or dashed lines, the structure or the portion of the structure is to be interpreted as encompassing all stereoisomers of it. Similarly, names of compounds having one or more chiral centers that do not specify the stereochemistry of those centers encompass pure stereoisomers and mixtures thereof. Moreover, any atom shown in a drawing with unsatisfied valences is assumed to be attached to enough hydrogen atoms to satisfy the valences.

Unless otherwise indicated, "an effective amount" of a compound is an amount sufficient to provide a therapeutic or positive benefit in the treatment or management of a disease, environment or condition, or to delay or minimize one or more symptoms associated with the disease, environment or condition. An effective amount of a compound is an amount of therapeutic agent, alone or in combination with other therapies, which provides a therapeutic benefit in the treatment or management of the disease, environment or condition. The term "effective amount" can encompass an amount that improves overall therapy, reduces or avoids symptoms or causes of a disease, environment or condition, or enhances the therapeutic efficacy of another therapeutic agent.

Unless otherwise indicated, the terms "treat," "treating" and "treatment" contemplate an action that occurs while a subject (such as patient) is suffering from the specified disease or disorder, which reduces the severity of the disease or disorder, or one or more of its symptoms, or retards or slows the progression of the disease or disorder.

The term "pharmaceutically acceptable salt" refers to those salts which are, within the scope of sound medical judgment, suitable for use in contact with the tissues of humans and lower animals without undue toxicity, irritation, and allergic response, and are commensurate with a reasonable benefit/risk ratio. Pharmaceutically acceptable salts are well known in the art. Pharmaceutically acceptable salts of the compounds of the present disclosure include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid, and perchloric acid or with organic acids such as acetic acid, oxalic acid, maleic acid, tartaric acid, citric acid, succinic acid, or malonic acid or by using other methods known in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, and valerate salts. Salts derived from appropriate bases include alkali metal, alkaline earth metal, ammonium and N⁺(C₁-C₄ alkyl) - 4 salts. Representative alkali or alkaline earth metal salts include sodium, lithium, potassium, calcium, and magnesium. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate, and aryl sulfonate.

The term "pharmaceutically acceptable carrier" refers to a carrier, whether diluent or excipient, that is compatible with the other ingredients of a formulation and not deleterious to the recipient thereof. Usable pharmaceutically acceptable carriers are disclosed in various references including Handbook of Pharmaceuticals Excipients edited by Raymond C Rowe, Paul J Sheskey, and Marian E Quinn. Pharmaceutically acceptable carrier can be selected from the group consisting of inert diluents, dispersing and/or granulating agents, surface active agents and/or emulsifiers, disintegrating agents, binding agents, preservatives, buffering agents, lubricating agents, and/or oils. Said compositions optionally further comprise at least one of additional biologically active compounds or agents.

The terms "administration" or "administering" a composition are defined to include providing the compound or the pharmaceutical composition of the present disclosure to the subject in need of treatment or control. In an alternative embodiment, said administering is conducted through oral, intravenous, intramuscular, cutaneous, subcutaneous, intrathecal, transdermal, implantation, sublingual, buccal, rectal, vaginal, ocular, otic, nasal, inhalation, or nebulization route.

Notwithstanding that the numerical ranges and parameters setting forth the broad scope of the present disclosure are approximations, the numerical values set forth in the specific examples are reported as precisely as possible. Any numerical value, however, inherently contains certain errors necessarily resulting from the standard deviation found in the respective testing measurements. Accordingly, unless indicated to the contrary, the numerical parameters set forth in the present disclosure and attached claims are approximations that can vary as desired. At the very least, each numerical parameter should at least be constructed in light of the number of reported significant digits and by applying ordinary rounding techniques

### Novel Compounds

The present disclosure provides novel compounds of peripheral alkyl and alkenyl chains extended benzene derivatives as defined in the claims. In some embodiments of the present disclosure, a compound represented by Formula (I-1), or its geometric isomers, enantiomers, diastereomers, racemates, pharmaceutically acceptable salts, and solvates thereof is provided.

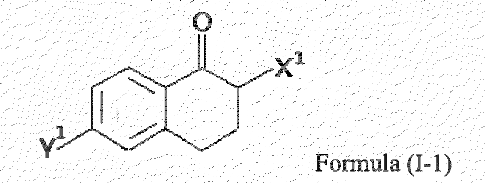

In Formula (I-1), X¹ is hydrogen, or R¹-A¹, and R¹ is a single bond or a double bond, A¹ is an aryl optionally substituted with -CF₃ groups; Y¹ is -NHC(O)R²C(O)NHR³, -R²C(O)NHR³, -R²C(O)NHR⁴C(O)NHR³, -C(O)NHR²C(O)NHR³, -OCH₂R⁵C(O)NHR³, -OCH₂R⁵R²C(O)NHR³ or -C(O)R⁶C(O)R²C(O)NHR³, wherein R² and R⁴ are independently selected from a group consisting of C₁-C₁₀ alkyl, C₁-C₁₀ alkenyl, and C₁-C₁₀ alkoxy, R³ is independently selected from a group consisting of -OH, an aryl optionally substituted with at least one of a halogen, an alkyl, an alkoxy or -NH₂, and when R³ is an aryl substituted with -NH₂, any hydrogen in the NH₂ may be optionally substituted with alkyl, R⁵ is an aryl and R⁶ is a heterocyclic alkyl. When Y¹ is OCH₂R⁵C(O)NHR³, R³ is an aryl optionally substituted with at least one of a halogen, an alkyl, an alkoxy or -NH₂.

In some embodiments, the compound of Formula (I-1) is selected from the group consisting of the compounds delineated in Table A or a geometric isomer, enantiomer, diastereomer, racemate, pharmaceutically acceptable salt, and solvate thereof.

**Table A:**

| (No.) | Structure | (No.) | Structure |
|---|---|---|---|
| 1 | 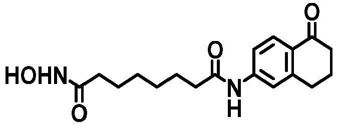 | | |
| 4 | 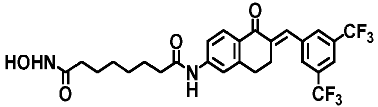 | 69 | 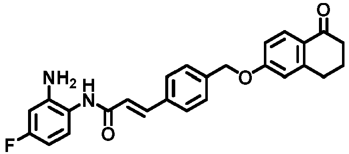 |
| 5 | 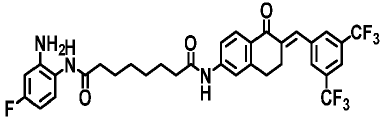 | 70 | 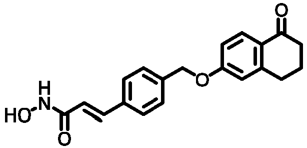 |
| 6 | 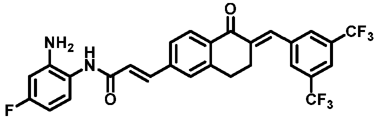 | 71 | 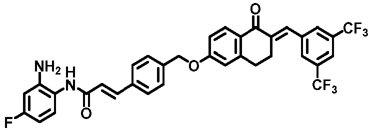 |
| 7 | 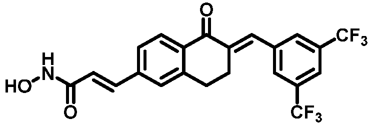 | 72 | 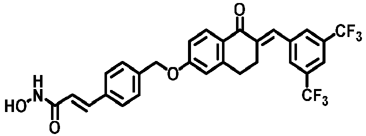 |
| 8 | 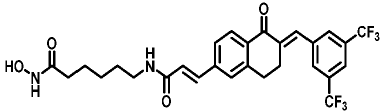 | 73 | 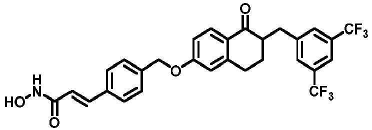 |
| 9 | 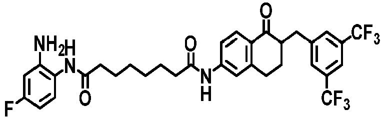 | 74 | 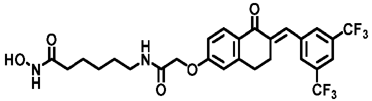 |
| 10 | 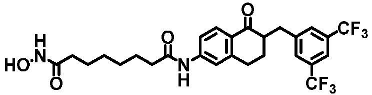 | 75 | 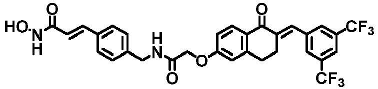 |
| 11 | 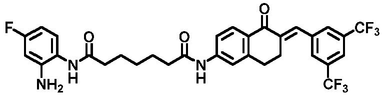 | 78 | 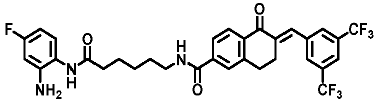 |
| 12 | 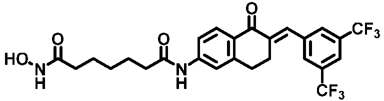 | 79 | 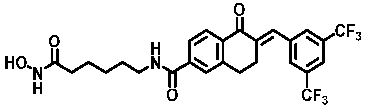 |
| 13 | 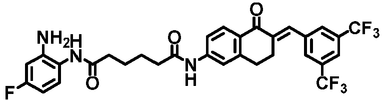 | 80 | 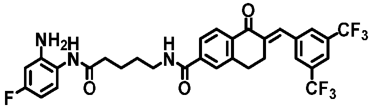 |
| 14 | 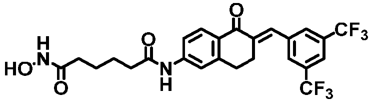 | 81 | 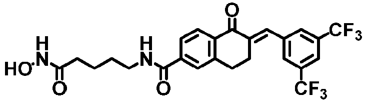 |
| 66 | 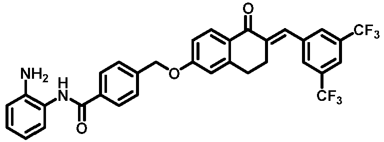 | 82 | 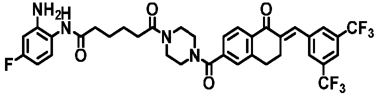 |
| 67 | 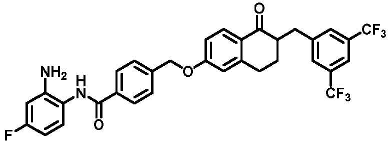 | 83 | 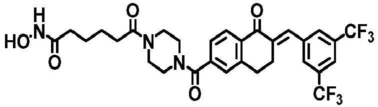 |

It will be appreciated that certain compounds of the above Formula (I-1) of the present disclosure can possess an asymmetric carbon atom(s) and are thus capable of existing as enantiomers. Unless otherwise specified, the present disclosure includes such enantiomers, including racemates. The separate enantiomers may be synthesized from chiral starting materials, or the racemates can be resolved by procedures that are well known in the art of chemistry such as chiral chromatography and fractional crystallization of diastereometric salts.

Active compounds of the present disclosure further comprise the use of the compounds according to Formula (I-1) for the prophylaxis and treatment of various diseases, disorders and/or conditions.

In another aspect, the present disclosure further provides a pharmaceutical composition comprising a therapeutically effective amount of the peripheral alkyl and alkenyl chains extended benzene derivative of Formula (I-1) and a pharmaceutically acceptable carrier.

The compounds of the present disclosure are useful as pharmaceutically active agents and may be utilized in bulk form. More preferably, these compounds are formulated into pharmaceutical formulations for administration. Any of a number of suitable pharmaceutical formulations may be utilized as a vehicle for the administration of the compounds of the present disclosure.

The compounds of the present disclosure may be formulated for administration for the treatment of a variety of conditions. In the manufacture of a pharmaceutical composition according to the present disclosure, the compounds of the present disclosure and the physiologically acceptable salts thereof, or the acid derivatives of either (hereinafter referred to as the "active compound") are typically admixed with, *inter alia,* an acceptable carrier. The carrier must, of course, be acceptable in the sense of being compatible with any other ingredients in the formulation and must not be deleterious to the patient. The carrier may be a solid or a liquid, or both, and is preferably formulated with the compound as a unit-dose formulation. One or more of each of the active compounds may be incorporated in the formulations of the present disclosure, which may be prepared by any of the well-known techniques of pharmacy consisting essentially of admixing the components, optionally including one or more accessory ingredients.

The present disclosure encompasses pharmaceutical compositions for the prophylaxis or the treatment of an inflammatory diseases, disorders and/or conditions. The pharmaceutical composition comprises a therapeutically effective amount of a compound according to Formula (I-1) of the present disclosure.

In one aspect of the present disclosure, the compounds according to Formula (I-1) are dual autotaxin / HDAC inhibitors. In some embodiments, the compounds according to the Formula (I-1) of the present disclosure can inhibit the activity of autotaxin in a cell. In some embodiments, the compounds according to the Formula (I-1) of the present disclosure can inhibit the activity of HDAC in a cell.

### Examples

### Example I: Synthesis of Compounds 1-83

To prove that the compounds of the present disclosure may be appropriately synthesized and utilized, the synthesis of the Compounds delineated in Tables A are described in detail below.

### Synthesis of Compound 1

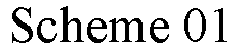

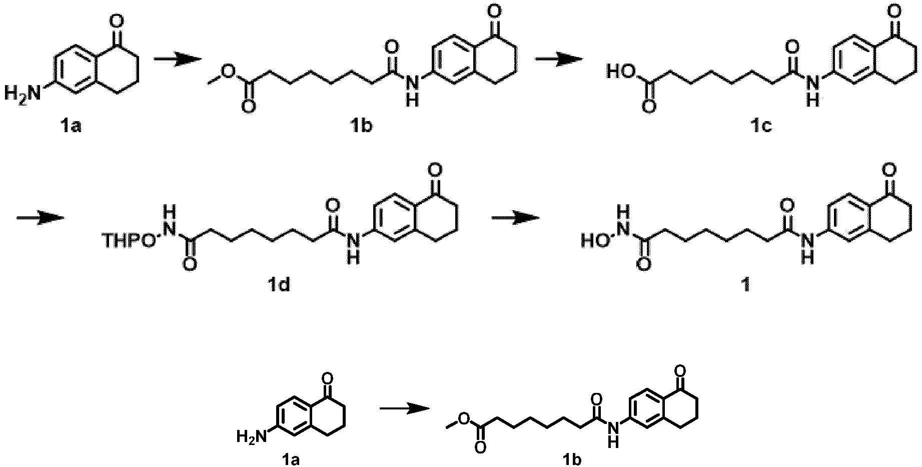

To a solution of Compound **1a** (6-Amino-1-tetralone) (0.50 g, 3.10 mmol), suberic acid monomethyl ester (0.70 g, 3.72 mmol) and hydroxybenzotriazole (HOBt; 0.21 g, 1.55 mmol) in dichloromethane (DCM; 50 mL) was added N,N-diisopropylethylamine (DIPEA; 1.20 g, 9.31 mmol) and 1-ethyl-3-(3-dimethylaminopropyl)carbodiimide (EDCI; 0.82 g, 4.65 mmol) at 0°C. After addition, the reaction mixture was slowly warmed to room temperature (RT) and stirred for overnight.

After reaction was completed, the solvent was removed under reduced pressure. The residual was diluted with EtOAc and washed with *Sat.* (saturated) NH₄Cl and *Sat.* NaHCO₃. The combined organic layers were washed with brine and dried over MgSO₄ and concentrated in vacuo to afford Compound **1b** (methyl 8-oxo-8-((5-oxo-5,6,7,8-tetrahydronaphthalen-2-yl)amino)octanoate) (0.44 g, 1.33 mmol, yield 43%). The product (Compound **1b**) was used in next step without further purification.

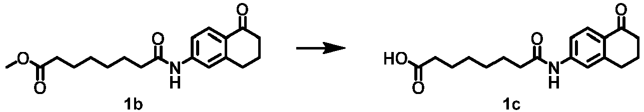

To a solution of Compound **1b** (0.44 g, 1.33 mmol) in MeOH (20 mL) was added 2N NaOH (2 mL, 4.00 mmol). After addition, the reaction mixture was stirred for overnight.

After reaction was completed, the solvent was removed under reduced pressure. The residual was diluted with ice-cold water and acidified with 1N HCl. The precipitated solid was collected by filtration and washed with 50% MeOH/water to give Compound **1c** (8-oxo-8-((5-oxo-5,6,7,8-tetrahydronaphthalen-2-yl)amino)octanoic acid) (0.36 g, 1.13 mmol, yield 85%). The product (Compound **1c**) was used in next step without further purification.

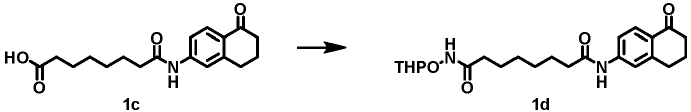

To a solution of Compound **1c** (0.1 g, 0.32 mmol) and O-(Tetrahydro-2H-pyran-2-yl)hydroxylamine (0.06 g, 0.47 mmol) in dimethylformamide (DMF; 10 mL) was added EDCI (0.09 g, 0.47 mmol) at 0°C. After addition, the reaction mixture was slowly warmed to RT and stirred for overnight.

After reaction was completed, the reaction mixture was diluted with EtOAc and washed with *Sat.* NH₄Cl. The combined organic layers were washed with brine and dried over MgSO₄ and concentrated in vacuo. The crude product was purified via flash column chromatography on a silica gel column using 10:1 DCM-MeOH as the eluent to give Compound **1d** (*N¹*-(5-oxo-5,6,7,8-tetrahydronaphthalen-2-yl)-*N⁸*-((tetrahydro-2H-pyran-2-yl)oxy)octanediamid e) (0.06 g, 0.14 mmol, yield 45%).

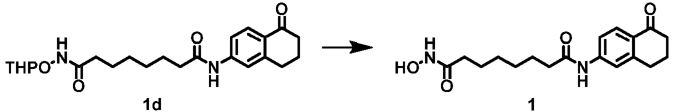

To a solution of Compound **1d** (0.06 g, 0.14 mmol) in MeOH (10 mL) was added 1N HCl (0.5 mL) and stirred for 3 hrs.

After reaction was completed, the solvent was removed under reduced pressure. The precipitated solid was washed with water and ether to produce Compound **1** (*N¹*-hydroxy-*N⁸*-(5-oxo-5,6,7,8-tetrahydronaphthalen-2-yl)octanediamide) (0.02 g, 0.05 mmol, yield 39%).

Compound **1,** ¹H-NMR (400 MHz, CD₃OD): δ 7.91-7.89 (d, 1H), 7.64 (s, 1H), 7.46-7.45 (d, 1H), 2.96-2.94 (m, 2H), 2.62-2.59 (m, 2H), 2.41-2.38 (m, 2H), 2.12-2.08 (m, 4H), 1.70-1.62 (m, 4H), 1.40-1.39 (m, 4H). ESI-MS *m*/*z* calcd for C₁₈H₂₄N₂O₄ 332.17, found 355 [M+Na]⁺.

### Synthesis of Compounds 4~5

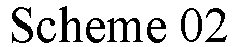

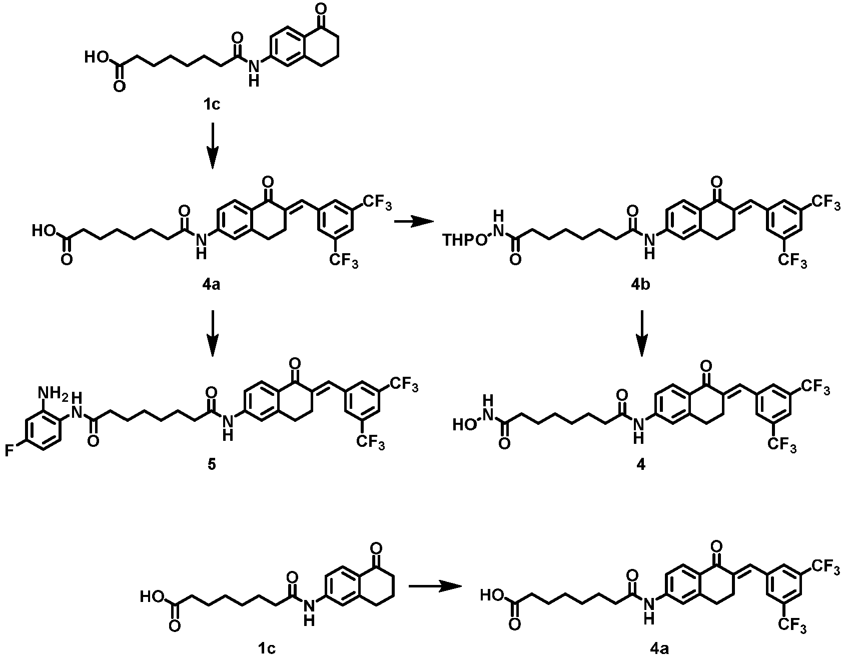

To a solution of Compound **1c** (0.1 g, 0.32 mmol) and 3,5-Bis(trifluoromethyl)benzaldehyde (0.09 g, 0.38 mmol) in MeOH (10 mL) was added 2N NaOH (1 mL, 2.00 mmol). After addition, the reaction mixture was stirred for overnight.

After reaction was completed, the solvent was removed under reduced pressure. The residual was diluted with ice-cold water and acidified with 1N HCl. The precipitated solid was collected by filtration and washed with 50% MeOH/water to get Compound **4a** ((*E*)-8-((6-(3,5-bis(trifluoromethyl)benzylidene)-5-oxo-5,6,7,8-tetrahydronaphthalen-2-yl)amino )-8-oxooctanoic acid) (0.13 g, 0.24 mmol , yield 75%).

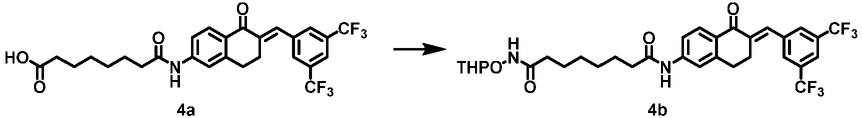

To a solution of Compound **4a** (0.13 g, 0.24 mmol) and O-(Tetrahydro-2H-pyran-2-yl)hydroxylamine (0.04 g, 0.36 mmol) in DMF (10 mL) was added EDCI (0.07 g, 0.36 mmol) at 0°C. After addition, the reaction mixture was slowly warmed to RT and stirred for overnight.

After reaction was completed, the reaction mixture was diluted with EtOAc and washed with *Sat.* NH₄Cl. The combined organic layers were washed with brine and dried over MgSO₄ and concentrated in vacuo. The crude product was purified via flash column chromatography on a silica gel column using 10:1 DCM-MeOH as the eluent to give Compound **4b** ((*E*)-*N¹*-(6-(3,5-bis(trifluoromethyl)benzylidene)-5-oxo-5,6,7,8-tetrahydronaphthalen-2-yl)-*N⁸*-((t etrahydro-2H-pyran-2-yl)oxy)octanediamide) (0.10 g, 0.16 mmol, yield 67%).

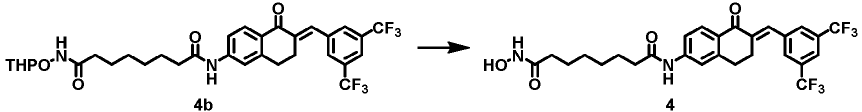

To a solution of Compound **4b** (0.10 g, 0.16 mmol) in MeOH (10 mL) was added 1N HCl (0.5 mL) and stirred for 2 hrs.

After reaction was completed, the solvent was removed under reduced pressure. The precipitated solid was washed with water and ether to yield Compound **4** ((*E*)-*N¹*-(6-(3,5-bis(trifluoromethyl)benzylidene)-5-oxo-5,6,7,8-tetrahydronaphthalen-2-yl)-*N⁸*-h ydroxyoctanediamide) (0.08 g, 0.14 mmol, yield 89%).

Compound **4,** ¹H-NMR (400 MHz, CD₃OD): δ 7.31-7.29 (d, 2H), 6.94-6.93 (d, 1H), 6.43-6.41 (d, 1H), 5.98-5.94 (m, 1H), 2.76-2.73 (m, 2H), 2.37-2.34 (m, 2H), 2.30-2.26 (m, 2H), 2.11-2.08 (m, 2H), 1.71-1.62 (m, 5H), 1.40-1.39 (m, 5H). ESI-MS *m*/*z* calcd for C₂₇H₂₆F₆N₂O₄ 556.18, found 557 [M+H]⁺.

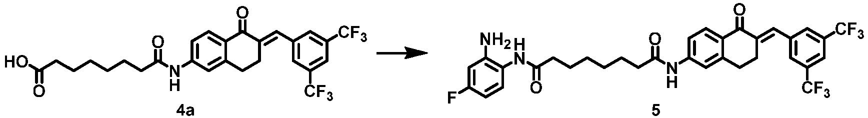

To a solution of Compound **4a** (0.2 g, 0.37 mmol), 4-fluoro-1,2-phenylenediamine (0.06 g, 0.44 mmol) and 4-dimethylaminopyridine (DMAP; 0.05 g, 0.37 mmol) in THF (20 mL) was added NMM (0.06 g, 0.56 mmol) and EDCI (0.11 g, 0.55 mmol) at 0 °C. After addition, the reaction mixture was warmed to room temperature and stirred for overnight.

After reaction was completed, the reaction mixture was diluted with EtOAc and washed with *Sat.* NH₄Cl. The combined organic layers were washed with brine and dried over MgSO₄ and concentrated in vacuo. The crude product was purified via flash column chromatography on a silica gel column using 2:1 EtOAc-Hexane as the eluent to give compound **5** ((E)-N 1-(2-amino-4-fluorophenyl)-N8-(6-(3, 5-bis(trifluoromethyl)benzylidene)-5-oxo-5,6,7, 8-te trahydronaphthalen-2-yl)octanediamide) (0.09 g, 0.14 mmol, yield 39%).

Compound **5,** ¹H-NMR (500 MHz, d₆-DMSO): δ 10.25(s, 1H), 9.01(s, 1H), 8.18(s, 2H), 8.12(s, 1H), 7.95-7.93(d, 1H), 7.77(s, 1H), 7.69(s, 1H), 7.58-7.56(d, 1H), 7.10-7.07(dd, 1H), 6.49-6.46(dd, 1H), 6.30-6.27(td, 1H), 5.12(s, 2H), 3.04-2.90(m, 4H), 2.38-2.35(t, 2H), 2.31-2.28(t, 2H), 1.60(br, 4H), 1.34(br, 4H). ESI-MS *m*/*z* calcd for C₃₃H₃₀F₇N₃O₃ 649.22, found 650 [M+H]⁺.

### Synthesis of Compounds 6~7

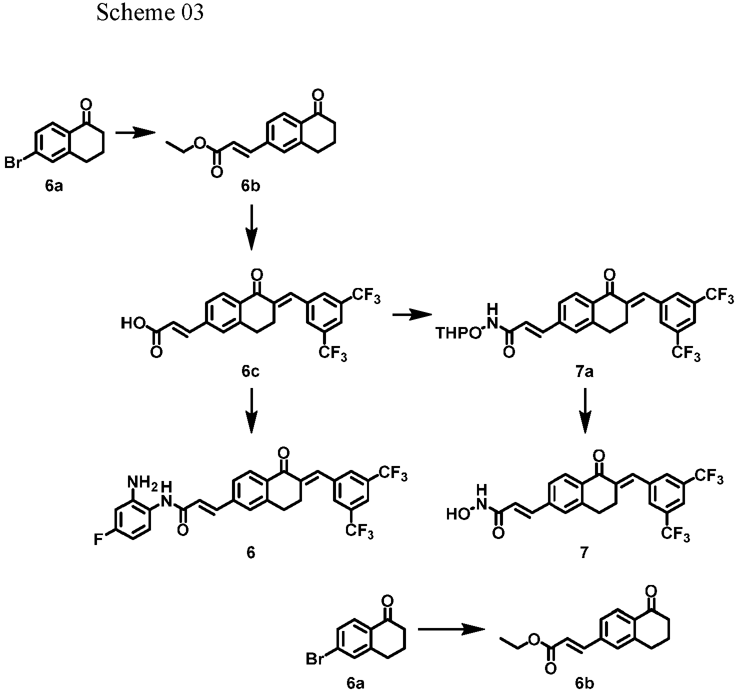

To a solution of compound **6a** (6-bromo-3,4-dihydronaphthalen-1(2H)-one) (220 mg, 1.0 eq) in DMF (0.1 M) was added ethyl acrylate (1.3 eq) at rt, followed Et₃N (0.05 M), PPh₃ (0.4 eq). The mixture was stirred at RT for 5 min then degas with nitrogen, followed added Pd(OAc)₂ (0.05 eq). The reaction was stirred at 100 °C for overnight.

After cooling to RT, removed the solvent in vacuo and purified with column chromatography to afford the compound **6b** (ethyl (E)-3-(5-oxo-5,6,7,8-tetrahydronaphthalen-2-yl)acrylate) (179 mg, yield 75%).

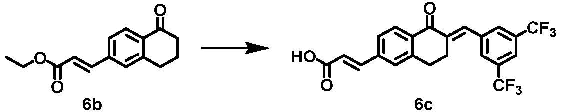

To a solution of compound **6b** (ethyl (E)-3-(5-oxo-5,6,7,8-tetrahydronaphthalen-2-yl)acrylate) (280 mg, 1 eq) in MeOH (10 mL) was added 3,5-bis(trifluoromethyl)benzaldehyde (333 mg, 1 eq), NaOH aqueous solution (1.0 M, 2.2 mL) at RT.

The mixture was stirred at RT for overnight, then removed the solvent in vacuo followed quench with HCl aqueous solution (1.0 M) to pH = 3. The mixture was extracted with EtOAc, then take the organic layer washed with brine. The combined organic layer was dried over anhydrous MgSO₄. Remove the solvent to afford the compound **6c** ((E)-3-(6-((E)-3,5-bis(trifluoromethyl)benzylidene)-5-oxo-5,6,7,8-tetrahydronaphthalen-2-yl)acr ylic acid) (414 mg, yield 82%).

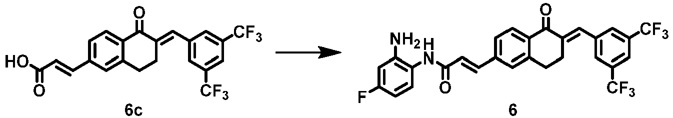

To a solution of compound **6c** ((E)-3-(6-((E)-3,5-bis(trifluoromethyl)benzylidene)-5-oxo-5,6,7,8-tetrahydronaphthalen-2-yl)acr ylic acid) (140 mg, 1 eq) in tetrahydrofuran (THF; 0.1 M) at 0 °C under nitrogen was added EDC hydrochloride (91 mg, 1.5 eq), followed N-methylmorpholine (NMM; 48 mg, 1.5 eq), HOBT (19 mg, 0.4 eq) and 4-Fluoro-1,2-phenylenediamine (48 mg, 1.2 eq). The mixture was allowed to warm to RT and stirred for overnight.

After removing the solvent, the crude product was purified by column chromatography to afford the compound **6** ((E)-N-(2-amino-4-fluorophenyl)-3-(6-((E)-3,5-bis(trifluoromethyl)benzylidene)-5-oxo-5,6,7,8-t etrahydronaphthalen-2-yl)acrylamide) (5 mg, yield 3%).

Compound **6,** ¹H-NMR (500 MHz, CD₃OD): δ 8.12-8.10 (d, 1H), 8.05 (s, 2H), 7.99 (s, 1H), 7.87 (s, 1H), 7.71-7.65 (m, 2H), 7.58 (s, 1H), 7.19 (m, 1H), 6.98-6.95 (d, 1H), 6.59-6.56 (dd, 1H), 6.43-6.40 (td, 1H), 3.13-3.06 (m, 4H). ESI-MS *m*/*z* calcd for C₂₈H₁₉F₇N₂O₂ 548.13, found 549 [M+H]⁺.

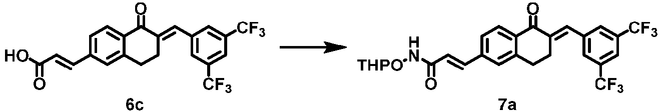

To a solution of compound **6c** ((E)-3-(6-((E)-3,5-bis(trifluoromethyl)benzylidene)-5-oxo-5,6,7,8-tetrahydronaphthalen-2-yl)acr ylic acid) (300 mg, 1.0 eq) in THF at 0 °C under nitrogen was added EDC hydrochloride (156 mg, 1.2 eq), followed NMM (83 mg, 1.2 eq), HOBT (42 mg, 0.4 eq) and *O*-(Tetrahydro-2H-pyran-2-yl)hydroxylamine (120 mg, 1.5 eq). The mixture was allowed to warm to RT and stirred for overnight.

After removing the solvent, the crude product was purified by column chromatography to afford compound **7a** ((E)-3-(6-((E)-3,5-bis(trifluoromethyl)benzylidene)-5-oxo-5,6,7,8-tetrahydronaphthalen-2-yl)-N-((tetrahydro-2H-pyran-2-yl)oxy)acrylamide) (300 mg, yield 82%).

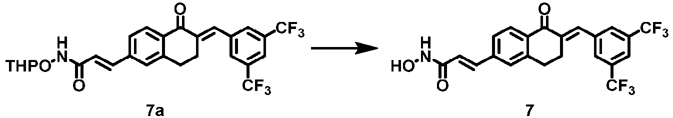

To a solution of compound **7a** ((E)-3-(6-((E)-3,5-bis(trifluoromethyl)benzylidene)-5-oxo-5,6,7,8-tetrahydronaphthalen-2-yl)-N-((tetrahydro-2H-pyran-2-yl)oxy)acrylamide) (200 mg, 1.0 eq) in MeOH (3.8 mL) at RT was added HCl aqueous solution (1.0 M, 1mL).

The reaction was stirred at RT for 2 hours, then the solid was filtered out to afford the compound **7**
((E)-3-(6-((E)-3,5-bis(trifluoromethyl)benzylidene)-5-oxo-5,6,7,8-tetrahydronaphthalen-2-yl)-N-hydroxyacrylamide) (20 mg, yield 8%).

Compound **7,** ¹H-NMR (500 MHz, d₆-DMSO): δ 8.19 (s, 2H), 8.13 (s, 1H), 8.01-7.99 (d, 1H), 7.82 (s, 1H), 7.62-7.60 (d, 1H), 7.58 (s, 1H), 7.51-7.48 (d, 1H), 6.63-6.60 (d, 1H), 3.07-2.98 (m, 4H). ESI-MS *m*/*z* calcd for C₂₂H₁₅F₆NO₃ 455.10, found 456 [M+H]⁺.

### Synthesis of Compound 8

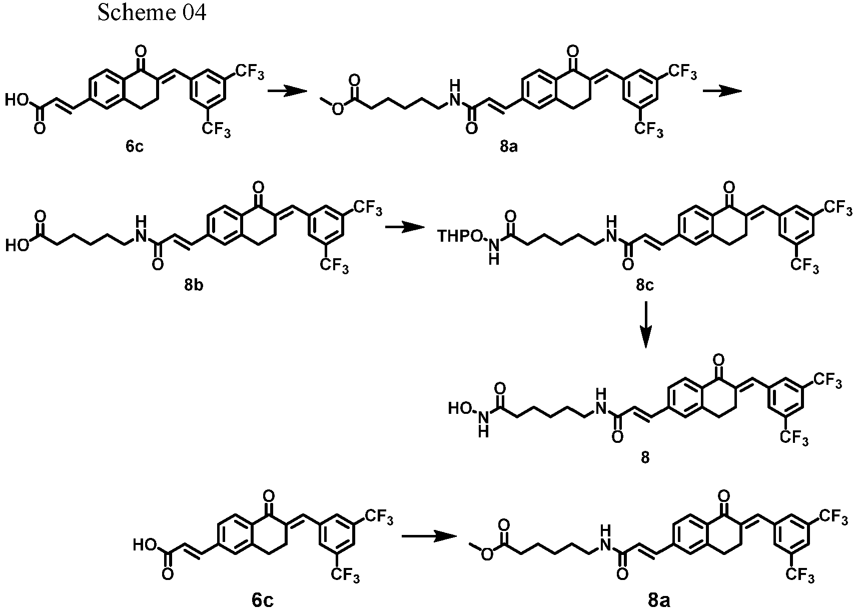

To a solution of compound **6c** ((E)-3-(6-((E)-3,5-bis(trifluoromethyl)benzylidene)-5-oxo-5,6,7,8-tetrahydronaphthalen-2-yl)acr ylic acid) (300 mg, 1.0 eq) in THF at 0 °C under nitrogen was added EDC hydrochloride (156 mg, 1.2 eq), followed NMM (82 mg, 1.2 eq), HOBT (42 mg, 0.4 eq) and methyl 6-aminohexanoate (162 mg, 1.5 eq). The mixture was allowed to warm to RT and stirred for overnight.

After removing the solvent, the crude product was purified by column chromatography to afford the product **8a** (methyl 6-((E)-3-(6-((E)-3,5-bis(trifluoromethyl)benzylidene)-5-oxo-5,6,7,8-tetrahydronaphthalen-2-yl)a crylamido)hexanoate) (367 mg, yield 95%).

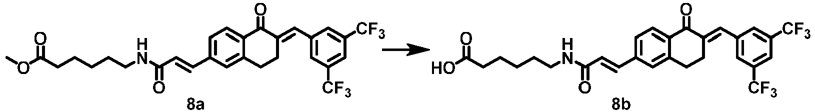

To a solution of compound **8a** (methyl 6-((E)-3-(6-((E)-3,5-bis(trifluoromethyl)benzylidene)-5-oxo-5,6,7,8-tetrahydronaphthalen-2-yl)a crylamido)hexanoate) (367 mg, 0.65 mmol) in MeOH (50 mL) was added 2N NaOH (1 mL) and stirred for overnight.

After reaction was completed, the solvent was removed under reduced pressure. The residual was diluted with ice-cold water and acidified with 1N HCl. The precipitated solid was collected by filtration and washed with 30% MeOH/water to get compound **8b** (6-((E)-3-(6-((E)-3, 5-bis(trifluoromethyl)benzylidene)-5-oxo-5,6, 7, 8-tetrahydronaphthalen-2-yl) acrylamido)hexanoic acid) (288 mg, 0.52 mmol , yield 80%).

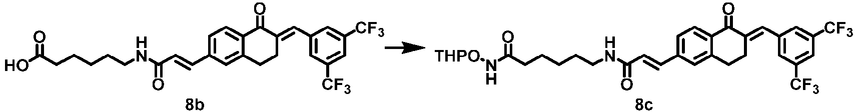

To a solution of compound **8b** (6-((E)-3-(6-((E)-3,5-bis(trifluoromethyl)benzylidene)-5-oxo-5,6,7,8-tetrahydronaphthalen-2-yl) acrylamido)hexanoic acid) (230 mg, 1.0 eq) in THF (0.1 M) at 0 °C under nitrogen was added EDC hydrochloride (99 mg, 1.2 eq), followed NMM (52 mg, 1.2 eq), HOBT (26 mg, 0.4 eq) and O-(Tetrahydro-2H-pyran-2-yl)hydroxylamine (75 mg, 1.5 eq). The mixture was allowed to warm to RT and stirred for overnight.

After removing the solvent, the crude product was purified by column chromatography to afford the product **8c** (6-((E)-3-(6-((E)-3,5-bis(trifluoromethyl)benzylidene)-5-oxo-5,6,7,8-tetrahydronaphthalen-2-yl) acrylamido)-N-((tetrahydro-2H-pyran-2-yl)oxy)hexanamide) (130 mg, yield 48%).

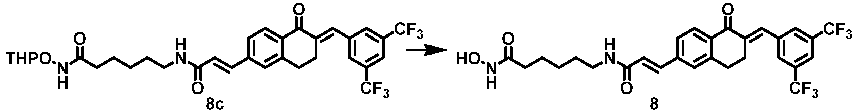

To a solution of compound **8c** (6-((E)-3-(6-((E)-3,5-bis(trifluoromethyl)benzylidene)-5-oxo-5,6,7,8-tetrahydronaphthalen-2-yl) acrylamido)-N-((tetrahydro-2H-pyran-2-yl)oxy)hexanamide) (130 mg, 1.0 eq) was added HCl aqueous solution (1.0 M, 0.6 mL).

The reaction was stirred at RT for 2 hours, then the solid was filtered out to afford the product **8** (6-((E)-3-(6-((E)-3,5-bis(trifluoromethyl)benzylidene)-5-oxo-5,6,7,8-tetrahydronaphthalen-2-yl) acrylamido)-N-hydroxyhexanamide) (30 mg, yield 26%).

Compound **8,** ¹H-NMR (500 MHz, d₆-DMSO): δ 10.34 (s, 1H), 10.16 (s, 1H), 8.20 (s, 2H), 8.14 (s, 1H), 8.02-8.00 (d, 1H), 7.83 (s, 1H), 7.61-7.60 (d, 1H), 7.56 (s, 1H), 7.46-7.43 (d, 1H), 6.79-6.76 (d, 1H), 3.17-3.16 (m, 2H), 3.08-3.07 (m, 1H), 3.00-2.99 (m, 1H), 1.96-1.93 (t, 2H), 1.52-1.44 (m, 4H), 1.28-1.27 (m, 2H). ESI-MS *m*/*z* calcd for C₂₈H₂₆F₆N₂O₄ 568.18, found 569 [M+H]⁺.

### Synthesis of Compounds 9~10

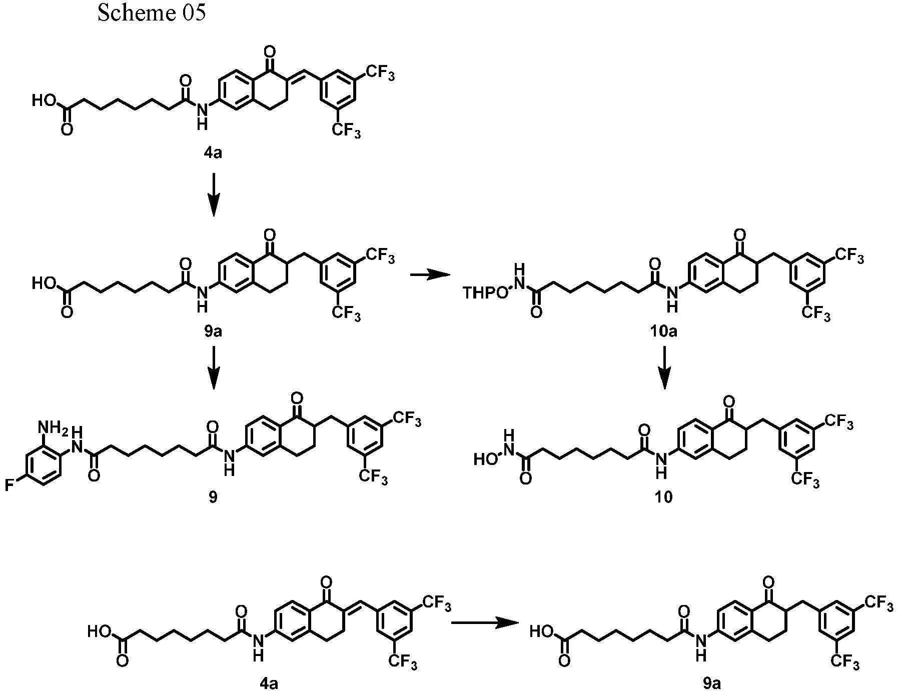

To a solution of Compound **4a** ((*E*)-8-((6-(3,5-bis(trifluoromethyl)benzylidene)-5-oxo-5,6,7,8-tetrahydronaphthalen-2-yl)amino )-8-oxooctanoic acid) (0.20 g, 0.37 mmol) in MeOH (50 mL) was added Pd/C (0.1 g). After addition, the reaction mixture was stirred under H₂ for 30 mins.

After reaction was completed, the solvent was removed under reduced pressure. Pd/C was filed off through a pad of celite and washed with EtOAc. The filtrate was concentrated in vacuo to afford compound **9a** (8-((6-(3,5-bis(trifluoromethyl)benzyl)-5-oxo-5,6,7,8-tetrahydronaphthalen-2-yl)amino)-8-oxooc tanoic acid) (0.17 g, 0.31 mmol, yield 83%).

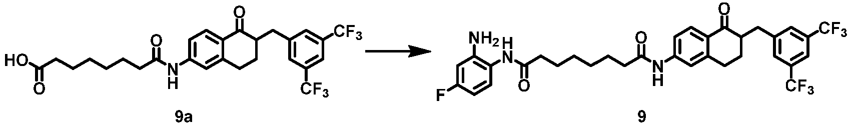

To a solution of compound **9a** (8-((6-(3,5-bis(trifluoromethyl)benzyl)-5-oxo-5,6,7,8-tetrahydronaphthalen-2-yl)amino)-8-oxooc tanoic acid) (0.47 g, 0.86 mmol), 4-fluoro-1,2-phenylenediamine (0.12 g, 0.95 mmol) in DCM (30 mL) was added NMM (0.18 g, 1.73 mmol) and HATU (0.39 g, 1.04 mmol) at 0 °C. After addition, the reaction mixture was slowly warmed to room temperature and stirred for 3 hours.

After reaction was completed, the solvent was removed under reduced pressure. The residual was diluted with *Sat.* NH₄Cl. The precipitated solid was collected by filtration. The crude product was washed with 30% EtOAc/Hex to give compound **9** (N1-(2-amino-4-fluorophenyl)-N8-(6-(3,5-bis(trifluoromethyl)benzyl)-5-oxo-5,6,7,8-tetrahydron aphthalen-2-yl)octanediamide) (0.18 g, 0.28 mmol, yield 32%).

Compound **9,** ¹H-NMR (500 MHz, CD₃OD): δ 7.93-7.91(d, 1H), 7.89(s, 2H), 7.80(s, 1H), 7.62(s, 1H), 7.46-7.44(d, 1H), 7.02-6.99(dd, 1H), 6.54-6.51(dd, 1H), 6.37-6.33(td, 1H), 3.51-3.48(dd, 1H), 3.01-2.89(m, 4H), 2.42-2.39(t, 4H), 2.10-2.07(m, 1H), 1.82-1.78(m, 1H), 1.74-1.72(t, 4H), 1.46-1.45(t, 4H). ESI-MS *m*/*z* calcd for C₃₃H₃₂F₇N₃O₃ 651.23, found 652 [M+H]⁺.

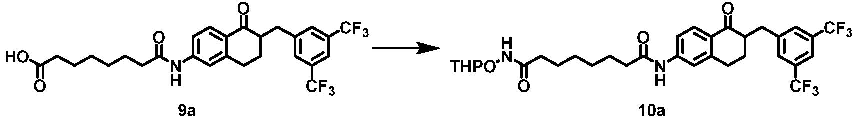

To a solution of compound **9a** (8-((6-(3,5-bis(trifluoromethyl)benzyl)-5-oxo-5,6,7,8-tetrahydronaphthalen-2-yl)amino)-8-oxooc tanoic acid) (0.17 g, 0.31 mmol), O-(Tetrahydro-2H-pyran-2-yl)hydroxylamine (0.04 g, 0.31 mmol) and DMAP (0.04 g, 0.31 mmol) in DCM (10 mL) was added NMM (0.03 g, 0.31 mmol) and EDCI (0.06 g, 0.31 mmol) at 0°C. After addition, the reaction mixture was slowly warmed to RT and stirred for overnight.

After reaction was completed, the reaction mixture was diluted with EtOAc and washed with *Sat.* NH₄Cl. The combined organic layers were washed with brine and dried over MgSO₄ and concentrated in vacuo. The crude product was purified via flash column chromatography on a silica gel column using 10:1 DCM-MeOH as the eluent to give compound **10a** (N1-(6-(3,5-bis(trifluoromethyl)benzyl)-5-oxo-5,6,7,8-tetrahydronaphthalen-2-yl)-N8-((tetrahydr o-2H-pyran-2-yl)oxy)octanediamide) (0.12 g, 0.19 mmol, yield 62%).

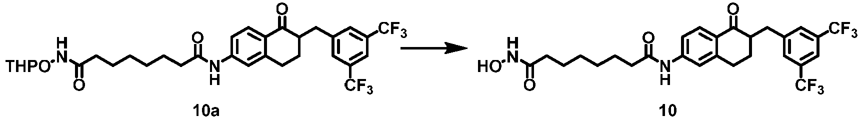

To a solution of compound **10a** (N1-(6-(3,5-bis(trifluoromethyl)benzyl)-5-oxo-5,6,7,8-tetrahydronaphthalen-2-yl)-N8-((tetrahydr o-2H-pyran-2-yl)oxy)octanediamide) (0.12 g, 0.19 mmol) in DCM (10 mL) was added 2N HCl (excess, in diethyl ether) and stirred for 3 hours.

After reaction was completed, the solvent was removed under reduced pressure. The precipitated solid was washed with water and ether to yield compound **10** (N1-(6-(3,5-bis(trifluoromethyl)benzyl)-5-oxo-5,6,7,8-tetrahydronaphthalen-2-yl)-N8-hydroxyoc tanediamide) (0.08 g, 0.01 mmol, yield 8%).

Compound **10,** ¹H-NMR (500 MHz, CD₃OD): δ 7.94-7.92(d, 1H), 7.89(s, 2H), 7.80(s, 1H), 7.62(s, 1H), 7.46-7.44(dd, 1H), 3.52-3.48(dd, 1H), 3.02-2.90(m, 4H), 2.40-2.37(t, 2H), 2.10-2.07(t, 2H), 1.85-1.79(m, 1H), 1.71-1.60(m, 5H), 1.39-1.38(m, 4H). ESI-MS *m*/*z* calcd for C₂₇H₂₃F₆N₂O₄ 558.20, found 559 [M+H]⁺.

### Synthesis of Compounds 11~12

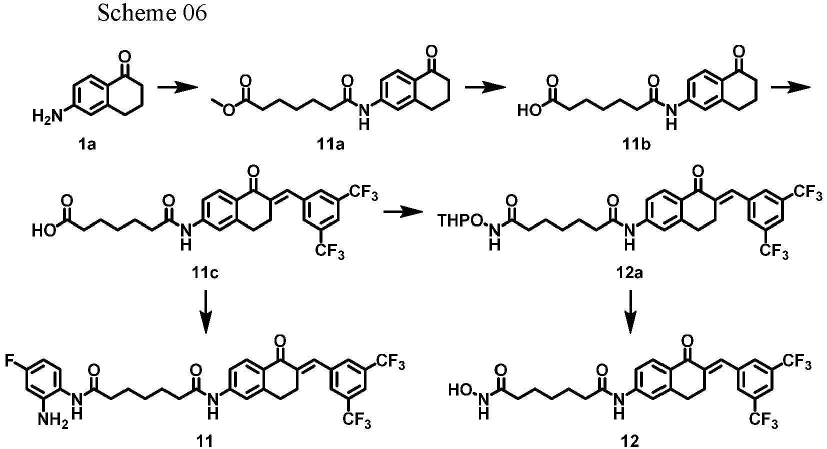

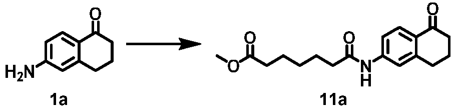

To a solution of Compound **1a** (6-Amino-1-tetralone) (0.50 g, 3.10 mmol), 7-methoxy-7-oxoheptanoic acid (0.81 g, 4.65 mmol) and HOBt (0.21 g, 1.55 mmol) in DCM (50 mL) was added DIPEA (1.20 g, 9.31 mmol) and EDCI (0.89 g, 4.65 mmol) at 0°C. After addition, the reaction mixture was slowly warmed to RT and stirred for overnight.

After reaction was completed, the solvent was removed under reduced pressure. The residual was diluted with EtOAc and washed with *Sat.* NH₄Cl and *Sat.* NaHCO₃. The combined organic layers were washed with brine and dried over MgSO₄ and concentrated in vacuo to afford Compound **11a** (methyl 7-oxo-7-((5-oxo-5,6,7,8-tetrahydronaphthalen-2-yl)amino)heptanoate) (0.88 g, 2.76 mmol, yield 89%). The product was used in next step without further purification.

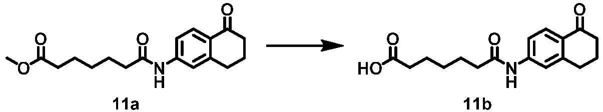

To a solution of compound **11a** (methyl 7-oxo-7-((5-oxo-5,6,7,8-tetrahydronaphthalen-2-yl)amino)heptanoate) (0.88 g, 2.76 mmol) in MeOH (50 mL) was added 2N NaOH (2 mL, 4.00 mmol). After addition, the reaction mixture was stirred for overnight.

After reaction was completed, the solvent was removed under reduced pressure. The residual was diluted with ice-cold water and acidified with 1N HCl. The precipitated solid was collected by filtration and washed with 30% MeOH/water to give Compound **11b** (7-oxo-7-((5-oxo-5,6,7,8-tetrahydronaphthalen-2-yl)amino)heptanoic acid) (0.78 g, 2.57 mmol, yield 93%). The product was used in next step without further purification.

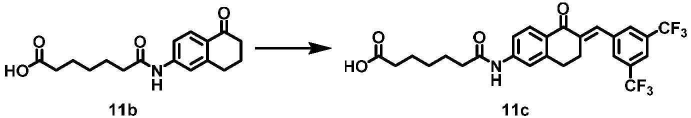

To a solution of Compound **11b** (7-oxo-7-((5-oxo-5,6,7,8-tetrahydronaphthalen-2-yl)amino)heptanoic acid) (0.67 g, 2.22 mmol) and 3,5-Bis(trifluoromethyl)benzaldehyde (0.65 g, 2.67 mmol) in MeOH (50 mL) was added 2N NaOH (3.30 mL, 6.67 mmol). After addition, the reaction mixture was stirred for overnight.

After reaction was completed, the solvent was removed under reduced pressure. The residual was diluted with ice-cold water and acidified with 1N HCl. The precipitated solid was collected by filtration and washed with 30% MeOH/water to get Compound **11c** ((E)-7-((6-(3,5-bis(trifluoromethyl)benzylidene)-5-oxo-5,6,7,8-tetrahydronaphthalen-2-yl)amino )-7-oxoheptanoic acid) (1.14 g, 2.16 mmol, yield 97%).

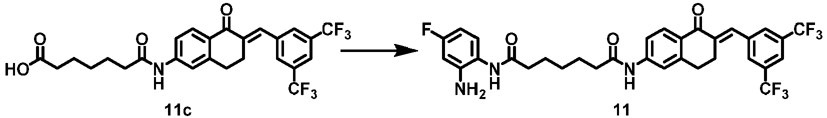

To a solution of Compound **11c** ((E)-7-((6-(3,5-bis(trifluoromethyl)benzylidene)-5-oxo-5,6,7,8-tetrahydronaphthalen-2-yl)amino )-7-oxoheptanoic acid) (0.66 g, 1.24 mmol), 4-fluoro-1,2-phenylenediamine (0.19 g, 1.49 mmol) and DMAP (0.08 g, 0.62 mmol) in THF (50 mL) was added NMM (0.19 g, 1.87 mmol) and EDCI (0.36 g, 1.87 mmol) at 0 °C. After addition, the reaction mixture was warmed to room temperature and stirred for overnight.

After reaction was completed, the reaction mixture was diluted with EtOAc and washed with *Sat.* NH₄Cl. The combined organic layers were washed with brine and dried over MgSO₄ and concentrated in vacuo. The crude product was purified via flash column chromatography on a silica gel column using 2:1 EtOAc-Hexane as the eluent to give Compound **11** ((E)-N1-(2-amino-4-fluorophenyl)-N7-(6-(3,5-bis(trifluoromethyl)benzylidene)-5-oxo-5,6,7,8-te trahydronaphthalen-2-yl)heptanediamide) (0.04 g, 0.07 mmol, yield 6%).

Compound 11, ¹H-NMR (500 MHz, CD₃OD): δ 8.02-8.00(m, 3H), 7.97(s, 1H), 7.81(s, 1H), 7.67(s, 1H), 7.54-7.52(d, 1H), 7.01-6.98(dd, 1H), 6.53-6.51(dd, 1H), 6.35-6.31(td, 1H), 3.08-2.95(m, 4H), 2.46-2.41(q, 4H), 1.79-1.76(t, 4H), 1.53-1.48(m, 2H). ESI-MS m/z calcd for C₃₂H₂₈F₇N₃O₃ 635.20, found 636 [M+H]⁺.

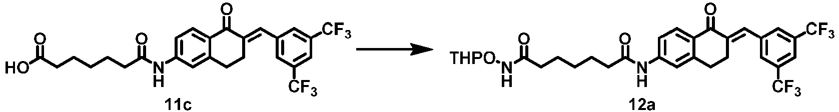

To a solution of Compound **11c** ((E)-7-((6-(3,5-bis(trifluoromethyl)benzylidene)-5-oxo-5,6,7,8-tetrahydronaphthalen-2-yl)amino )-7-oxoheptanoic acid) (0.49 g, 0.92 mmol), O-(Tetrahydro-2H-pyran-2-yl)hydroxylamine (0.04 g, 0.36 mmol), and DMAP (0.06 g, 0.47 mmol) in DCM (20 mL) was added EDCI (0.27 g, 1.38 mmol) at 0°C. After addition, the reaction mixture was slowly warmed to RT and stirred for overnight.

After reaction was completed, the reaction mixture was diluted with EtOAc and washed with *Sat.* NH₄Cl. The combined organic layers were washed with brine and dried over MgSO₄ and concentrated in vacuo. The crude product was purified via flash column chromatography on a silica gel column using 10:1 DCM-MeOH as the eluent to give Compound **12a** ((E)-N1-(6-(3,5-bis(trifluoromethyl)benzylidene)-5-oxo-5,6,7,8-tetrahydronaphthalen-2-yl)-N7-( (tetrahydro-2H-pyran-2-yl)oxy)heptanediamide) (0.22 g, 0.35 mmol, yield 38%).

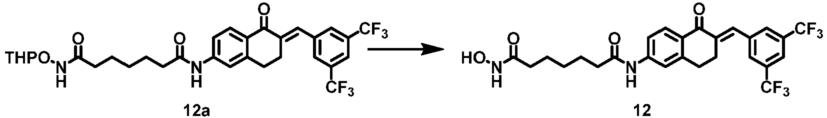

To a solution of Compound **12a** ((E)-N1-(6-(3,5-bis(trifluoromethyl)benzylidene)-5-oxo-5,6,7,8-tetrahydronaphthalen-2-yl)-N7-( (tetrahydro-2H-pyran-2-yl)oxy)heptanediamide) (0.22 g, 0.35 mmol) in MeOH (10 mL) was added 1N HCl (0.5 mL) and stirred for 2 hours.

After reaction was completed, the solvent was removed under reduced pressure. The precipitated solid was washed with water and ether to yield Compound **12** ((E)-N1-(6-(3,5-bis(trifluoromethyl)benzylidene)-5-oxo-5,6,7,8-tetrahydronaphthalen-2-yl)-N7-h ydroxyheptanediamide) (0.08 g, 0.14 mmol, yield 43%).

Compound **12,** ¹H-NMR (500 MHz, CD₃OD): δ 8.03-8.01(m, 3H), 7.97(s, 1H), 7.82(s, 1H), 7.69(s, 1H), 7.55-7.53(d, 1H), 3.09-2.98(m, 4H), 2.43-2.41(t, 2H), 2.13-2.10(t, 2H), 1.74-1.66(m, 4H), 1.43-1.40(m, 2H). ESI-MS *m*/*z* calcd for C₂₆H₂₄F₆N₂O₄ 542.16, found 565 [M+Na]⁺.

### Synthesis of Compounds 13~14

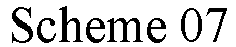

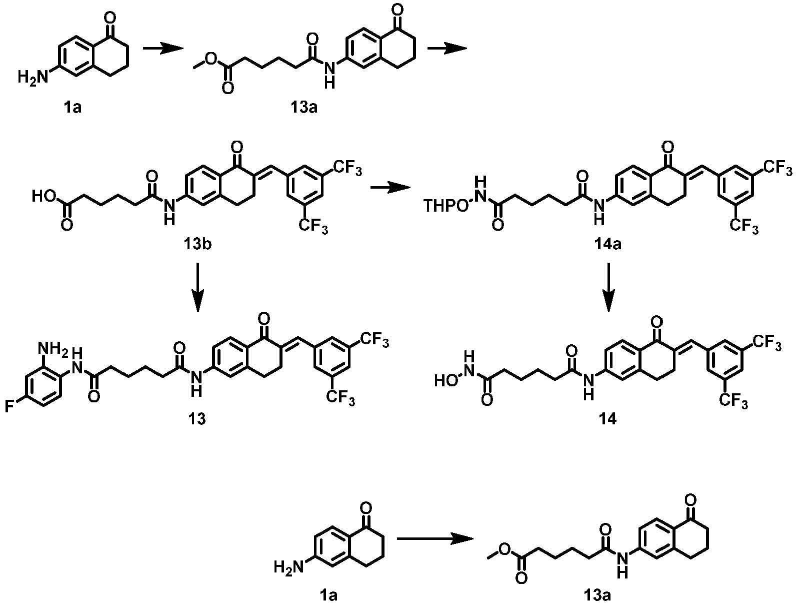

To a solution of Compound **1a** (6-Amino-1-tetralone) (1.00 g, 6.20 mmol), monomethyl adipate (1.29 g, 8.07 mmol) and DMAP (0.99 g, 8.07 mmol) in DCM (100 mL) was added DIPEA (2.41 g, 18.61 mmol) and EDCI (1.78 g, 9.31 mmol) at 0°C. After addition, the reaction mixture was slowly warmed to RT and stirred for overnight.

After reaction was completed, the solvent was removed under reduced pressure. The residual was diluted with EtOAc and washed with *Sat.* NH₄Cl and *Sat.* NaHCO₃. The combined organic layers were washed with brine and dried over MgSO₄ and concentrated in vacuo to afford Compound **13a** (methyl 6-oxo-6-((5-oxo-5,6,7,8-tetrahydronaphthalen-2-yl)amino)hexanoate) (1.48 g, 4.87 mmol, yield 78%). The product was used in next step without further purification.

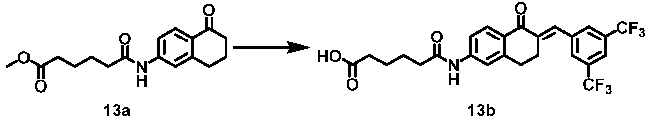

To a solution of Compound **13a** (methyl 6-oxo-6-((5-oxo-5,6,7,8-tetrahydronaphthalen-2-yl)amino)hexanoate) (1.12 g, 3.69 mmol) in MeOH (50 mL) was added 2N NaOH (4 mL, 8.00 mmol). After addition, the reaction mixture was stirred for overnight.

After the methyl ester was hydrolyzed, the reaction mixture was added 3,5-Bis(trifluoromethyl)benzaldehyde (1.07 g, 4.43 mmol) and stirred for further 6 hours. After the reaction was completed, the solvent was removed under reduced pressure. The residual was diluted with ice-cold water and acidified with 1N HCl. The precipitated solid was collected by filtration and washed with 30% MeOH/water to give Compound **13b** ((E)-6-((6-(3,5-bis(trifluoromethyl)benzylidene)-5-oxo-5,6,7,8-tetrahydronaphthalen-2-yl)amino )-6-oxohexanoic acid) (1.41 g, 2.75 mmol, yield 74%). The product was used in next step without further purification.

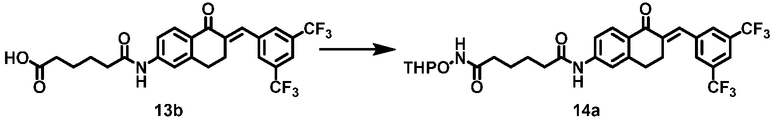

To a solution of Compound **13b** (E)-6-((6-(3,5-bis(trifluoromethyl)benzylidene)-5-oxo-5,6,7,8-tetrahydronaphthalen-2-yl)amino) -6-oxohexanoic acid (0.20 g, 0.39 mmol), O-(Tetrahydro-2H-pyran-2-yl)hydroxylamine (0.06 g, 0.51 mmol), and DMAP (0.02 g, 0.20 mmol) in DCM (20 mL) was added NMM (0.06 g, 0.59 mmol) and EDCI (0.11 g, 0.59 mmol) at 0°C. After addition, the reaction mixture was slowly warmed to RT and stirred for overnight.

After reaction was completed, the reaction mixture was diluted with EtOAc and washed with *Sat.* NH₄Cl. The combined organic layers were washed with brine and dried over MgSO₄ and concentrated in vacuo. The crude product was purified via flash column chromatography on a silica gel column using 10:1 DCM-MeOH as the eluent to give Compound **14a** ((E)-N1-(6-(3,5-bis(trifluoromethyl)benzylidene)-5-oxo-5,6,7,8-tetrahydronaphthalen-2-yl)-N6-( (tetrahydro-2H-pyran-2-yl)oxy)adipamide) (0.10 g, 0.16 mmol, yield 41%).

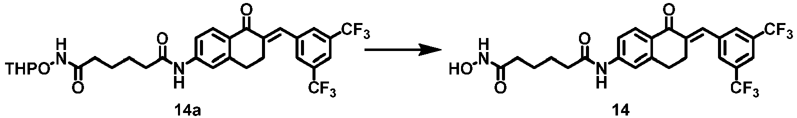

To a solution of Compound **14a** ((E)-N1-(6-(3,5-bis(trifluoromethyl)benzylidene)-5-oxo-5,6,7,8-tetrahydronaphthalen-2-yl)-N6-( (tetrahydro-2H-pyran-2-yl)oxy)adipamide) (0.10 g, 0.16 mmol) in DCM (10 mL) was added 2N HCl (excess, in diethyl ether) and stirred for 3 hrs.

After reaction was completed, the solvent was removed under reduced pressure. The precipitated solid was washed with water and ether to yield Compound **14** ((E)-N 1-(6-(3, 5-bis(trifluoromethyl)benzylidene)-5-oxo-5, 6,7, 8-tetrahydronaphthalen-2-yl)-N6-h ydroxyadipamide) (0.04 g, 0.07 mmol, yield 45%).

Compound **14,** ¹H-NMR (500 MHz, CD₃OD): δ 8.02-8.00(m, 3H), 7.97(s, 1H), 7.81(s, 1H), 7.67(s, 1H), 7.55-7.53(d, 1H), 3.08-3.07(t, 2H), 2.98-2.96(t, 2H), 2.44-2.41(t, 2H), 2.16-2.15(t, 2H), 1.70(s, 4H). ESI-MS *m*/*z* calcd for C₂₅H₂₂F₆N₂O₄ 528.15, found 529 [M+H]⁺.

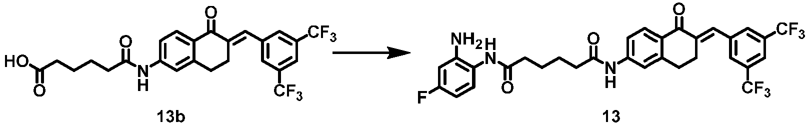

To a solution of Compound **13b** ((E)-6-((6-(3,5-bis(trifluoromethyl)benzylidene)-5-oxo-5,6,7,8-tetrahydronaphthalen-2-yl)amino )-6-oxohexanoic acid) (0.20 g, 0.39 mmol), 4-fluoro-1,2-phenylenediamine (0.05 g, 0.39 mmol) and DMAP (0.05 g, 0.39 mmol) in THF (50 mL) was added NMM (0.04 g, 0.39 mmol) and EDCI (0.10 g, 0.51 mmol) at 0 °C. After addition, the reaction mixture was warmed to room temperature and stirred for overnight.

After reaction was completed, the reaction mixture was diluted with EtOAc and washed with *Sat.* NH₄Cl. The combined organic layers were washed with brine and dried over MgSO₄ and concentrated in vacuo. The crude product was purified via flash column chromatography on a silica gel column using 2:1 EtOAc-Hexane as the eluent to give Compound **13** ((E)-N1-(2-amino-4-fluorophenyl)-N6-(6-(3,5-bis(trifluoromethyl)benzylidene)-5-oxo-5,6,7,8-te trahydronaphthalen-2-yl)adipamide) (0.11 g, 0.18 mmol, yield 47%).

Compound **13,** ¹H-NMR (500 MHz, CD₃OD): δ 8.03-8.01(m, 3H), 7.97(s, 1H), 7.82(s, 1H), 7.69(s, 1H), 7.56-7.54(dd, 1H), 7.04-7.01(m, 1H), 6.54-6.52(dd, 1H), 6.38-6.34(td, 1H), 3.10-3.08(t, 2H), 2.99-2.97(t, 2H), 2.49-2.45(m, 4H), 1.81-1.79(t, 4H). ESI-MS *m*/*z* calcd for C₃₁H₂₆F₇N₃O₃ 621.19, found 622 [M+H]⁺.

### Synthesis of Compound 66

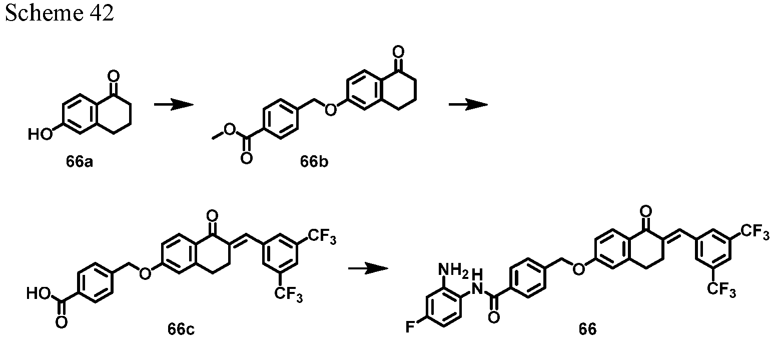

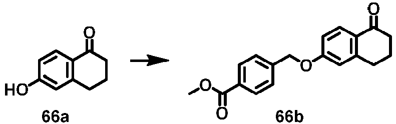

To a mixture solution of Compound **66a** (6-hydroxy-3,4-dihydronaphthalen-1(2H)-one) (0.7 g, 4.3 mmol), methyl 4-(bromomethyl)benzoate (1.09 g, 4.75 mmol) and K₂CO₃ (1.19 g, 8.6 mmol) in ACN (36 mL) was stirred at 60°C for overnight.

After cooling to RT, the crude was concentrated and the resulting material was washed with ether to provide the product as a solid product. Compound **66b** (methyl 4-(((5-oxo-5,6,7,8-tetrahydronaphthalen-2-yl)oxy)methyl)benzoate) (1.06 g, yield 80%).

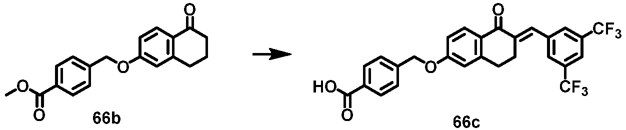

To a solution of Compound **66b** (methyl 4-(((5-oxo-5,6,7,8-tetrahydronaphthalen-2-yl)oxy)methyl)benzoate) (1.06 g, 3.4 mmol) in a co-solvent of MeOH:H₂O = 3:1 (15 mL) was added NaOH (0.26 g, 6.5 mmol) and stirred at 60°C for 3 hours.

The reaction mixture was concentrated to remove the organic solvent. The residue was diluted with water and acidified to pH 4 with 1N HCl_{(aq)}. The resulting precipitate was washed with H₂O and ether. The solid was further dried by pressing it on a filter paper.

Then treated the solid with 1N NaOH solution (1.7 mL) and and 3,5-bis(trifluoromethyl)benzaldehyde (98.0 mg, 0.41 mmol) in MeOH (3.4 mL) at RT for 24 hours.

The reaction mixture was concentrated to remove the organic solvent. The residue was diluted with water and acidified to pH = 4 with 1N HCl_{(aq)}. The resulting precipitate was filtered, washed with H₂O and MeOH, and dried by pressing it on a filter paper to obtain the white solid product Compound **66c** ((E)-4-(((6-(3, 5-bis(trifluoromethyl)benzylidene)-5-oxo-5,6,7, 8-tetrahydronaphthalen-2-yl)oxy) methyl)benzoic acid) (106.0 mg, yield 60%).

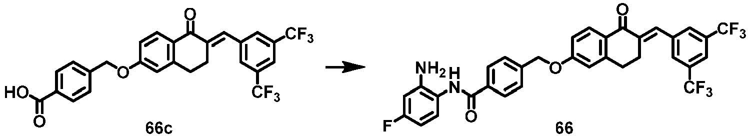

To a stirred solution of Compound **66c** ((E)-4-(((6-(3,5-bis(trifluoromethyl)-benzylidene)-5-oxo-5,6,7,8-tetrahydronaphthalen-2-yl)oxy) methyl)benzoic acid) (100.0 mg, 0.19 mmol) in DMF (1.9 mL) was added benzene-1,2-diamine (17.0 mg, 0.13 mmol) in one portion, followed by a solution of EDCI (22.0 mg, 0.11 mmol) and HOBt (31.0 mg, 2.30 mmol) in DMF (2.0 mL) was added dropwise to the mixture in one portion at 0°C. The resulting mixture was stirred at RT for overnight.

After which time the mixture concentrated in vacuo to give the crude material. The resulting residue was washed with ether and H₂O to provide the yellow solid product. Compound **66**
((E)-N-(2-aminophenyl)-4-(((6-(3,5-bis(trifluoromethyl)-benzylidene)-5-oxo-5,6,7,8-tetrahydron aphthalen-2-yl)oxy)methyl)benzamide)
(58.0 mg, yield 50%).

Compound **66,** ¹H-NMR (500 MHz, d₆-DMSO): δ 9.89 (s,1H), 8.16 (s,2H), 8.10 (s,1H), 8.01-7.96 (m,3H), 7.76 (s,1H), 7.60-7.58 (d,2H), 7.16-7.15 (d,1H), 7.08-7.06 (d,1H), 7.03 (s,1H), 6.99-6.96 (t,1H), 6.79-6.77 (d,1H), 6.62-6.59 (t,1H), 5.32 (s,1H), 4.88 (s,2H), 3.03 (s,2H), 2.93 (s,2H). ESI-MS *m*/*z* calcd for C₃₃H₂₄F₆N₂O₃ 610.17, found 611.3 [M+H]⁺.

### Synthesis of Compound 67

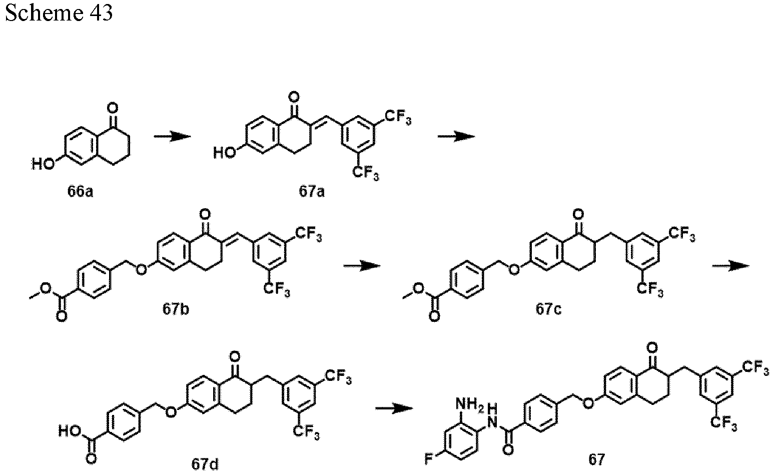

To a solution of Compound **66a** (6-Hydroxy-1-tetralone) (1311 mg, 8.00 mmol) in MeOH (60 mL) at RT was added hydrochloric acid (32%; 88 ml), followed 3,5-Bis(trifluoromethyl)benzaldehyde (2195 mg, 9.6 mmol).

The reaction was reflux for 12 hours, then ice-cold water was added and the resulting precipitate was collected by filtration, dried and purified by recrystallization with MeOH to afford the product Compound **67a** ((E)-2-(3,5-bis(trifluoromethyl)-benzylidene)-6-hydroxy-3,4-dihydronaphthalen-1(2H)-one) (1556 mg, 49%).

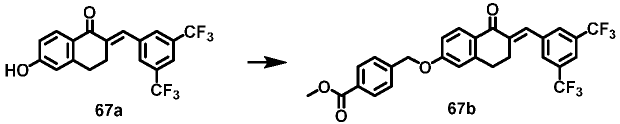

To a solution of Compound **67a** ((E)-2-(3,5-bis(trifluoromethyl)-benzylidene)-6-hydroxy-3,4-dihydronaphthalen-1(2H)-one) (386 mg, 1.00 mmol) in ACN (10 mL) at RT in pressure tube was added methyl 4-(bromomethyl)benzoate (233 mg, 1.00 mmol), Potassium carbonate (139 mg, 1.00 mmol). The tube was sealed and stirred at 95 °C for 90 mins.

After cooling to RT, removed the solvent in vacuo, the crude product was washed with water then recrystallization with ACN to afford the product Compound **67b** (methyl (E)-4-(((6-(3,5-bis(trifluoromethyl)benzylidene)-5-oxo-5,6,7,8-tetrahydronaphthalen-2-yl)oxy)m ethyl)benzoate) (330 mg, 62%).

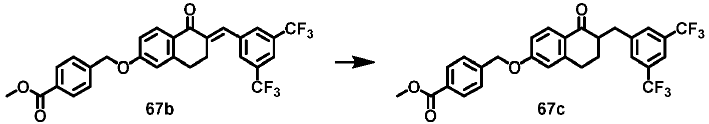

To a solution of Compound **67b** (methyl (E)-4-(((6-(3,5-bis(trifluoro-methyl)benzylidene)-5-oxo-5,6,7,8-tetrahydronaphthalen-2-yl)oxy) methyl)benzoate) (155 mg, 0.29 mmol) in DMSO (6 mL) at rt was added NH₄Cl (158 mg, 2.90 mmol), Zinc (99 mg, 1.45 mmol).

The mixture was sealed and stirred at RT for 11 hours, then diluted with EtOAc and washed with water followed brine. The combined organic layer was dried over anhydrous MgSO₄. After remove the solvent, the crude product was taken the next step. Compound **67c** (methyl
4-(((6-(3,5-bis(trifluoromethyl)benzyl)-5-oxo-5,6,7,8-tetrahydronaphthalen-2-yl)oxy)methyl)ben zoate) (145 mg, 93%).

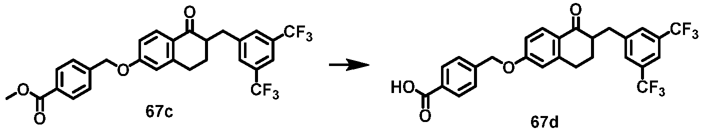

To a solution of Compound **67c** (methyl 4-(((6-(3,5-bis(trifluoromethyl)-benzyl)-5-oxo-5,6,7,8-tetrahydronaphthalen-2-yl)oxy)methyl)be nzoate) (386 mg, 0.72 mmol) in co-solvent MeOH (60 mL) and DCM (30 mL) was added NaOH aqueous solution (4.0 M, 5 mL) at RT.

The mixture was stirred at RT for 22 hours, then removed the solvent in vacuo followed quench with HCl aqueous solution (1.0 M) to pH = 4. The mixture was extracted with EtOAc, then take the organic layer washed with brine. The combined organic layer was dried over anhydrous MgSO₄. After remove the solvent, the crude product was taken the next step. Compound **67d** (4-(((6-(3,5-bis(trifluoromethyl)-benzyl)-5-oxo-5,6,7,8-tetrahydronaphthalen-2-yl)oxy)methyl)be nzoic acid) (265 mg, 70%).

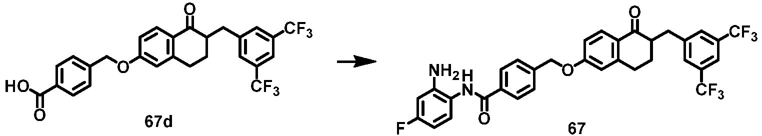

To a solution of Compound **67d** (4-(((6-(3,5-bis(trifluoromethyl)benzyl)-5-oxo-5,6,7,8-tetrahydronaphthalen-2-yl)oxy)methyl)be nzoic acid) (100 mg, 0.19 mmol) in DMF (30 mL) at 0 °C under nitrogen was added EDC hydrochloride (43 mg, 0.21 mmol), followed DMAP (27 mg, 0.21 mmol ) and 4-Fluoro-1,2-phenylenediamine (29 mg, 0.21 mmol). The mixture was allowed to warm to RT and stirred for 12 hours.

After removing the solvent, the crude product was purified by column chromatography (EtOAc:n-hexane = 1:2). The crude product was washed with MeOH, n-hexane to afford the product to afford the solid Compound **67** (*N*-(2-amino-4-fluorophenyl)-4-(((6-(3,5-bis(trifluoromethyl)benzyl)-5-oxo-5,6,7,8-tetrahydrona phthalen-2-yl)oxy)methyl)benzamide) (23 mg, 19%).

Compound **67,** ¹H-NMR (500 MHz, d₆-DMSO): δ 9.61 (s, 1H), 7.99 (s, 4H), 7.92 (s, 1H), 7.86-7.85 (d, 1H), 7.57-7.56 (d, 2H), 7.12-7.09 (t, 1H), 7.00-6.99 (d, 1H), 6.95 (s, 1H), 6.55-6.52 (dd, 1H), 6.38-6.34 (m, 1H), 5.28-5.22 (m, 4H), 3.15-2.87 (m, 4H), 1.98-1.95 (m, 1H), 1.75-1.68 (m, 1H). ESI-MS *m*/*z* calcd for C₃₃H₂₅F₇N₂O₃ 630.17, found 631.0 [M+H]⁺.

### Synthesis of Compounds 69~70

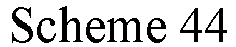

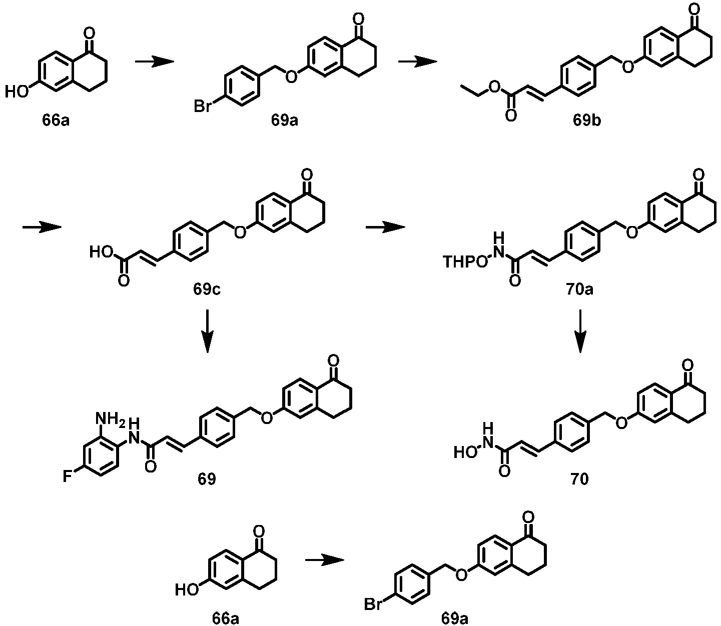

To a solution of Compound **66a** (6-Hydroxy-3,4-dihydro-1(2H)-naphthalenone) (1.00 g, 3.54 mmol) and K₂CO₃ (0.73 g, 5.31 mmol) in ACN (40 mL) was added 1-bromo-4-(bromomethyl)benzene (0.93 g, 3.72 mmol). After addition, the reaction mixture was heated to 50 °C and stirred for overnight.

The reaction was monitored by TLC. After reaction was completed, the solvent was removed under reduced pressure. The residual was diluted with ice-cold water and the precipitated solid was collected by filtration to get the desired product Compound **69a** (6-((4-bromobenzyl)oxy)-3,4-dihydronaphthalen-1(2H)-one)
(1.00 g, 3.01 mmol, yield 85%).

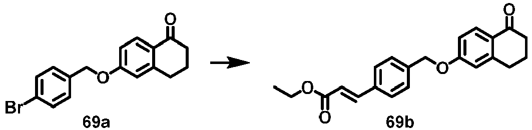

To a solution of Compound **69a** (6-((4-bromobenzyl)oxy)-3,4-dihydronaphthalen-1(2H)-one) (0.50 g, 1.51 mmol), triphenylphosphine (0.16 g, 0.60 mmol), ethyl acrylate (0.20 g, 1.96 mmol) in DMF/TEA (30 mL, 1:1) was added Pd(OAc)₂ (0.02 g, 0.08 mmol). After addition, the reaction mixture was heated to 100 °C and stirred for overnight.

The reaction was monitored by TLC. After reaction was completed, the reaction mixture was diluted with *sat.* NH₄Cl_{(aq)} and extracted with EtOAc. The organic phase was dried with MgSO₄ and concentrated under reduced pressure. The crude product was purified by silica gel column chromatography using ethyl acetate/hexane = 1/4 as elution to yield the desired product Compound **69b** (ethyl (E)-3-(4-(((5-oxo-5,6,7,8-tetrahydronaphthalen-2-yl)oxy)methyl)phenyl)acrylate) (0.51 g, 1.45 mmol, yield 96%).

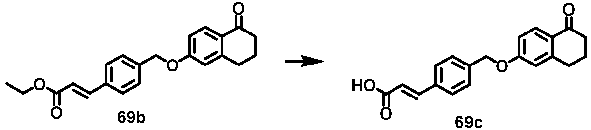

To a solution of Compound **69b** (ethyl (E)-3-(4-(((5-oxo-5,6,7,8-tetrahydronaphthalen-2-yl)oxy)methyl)phenyl)acrylate) (0.51 g, 1.45 mmol) in MeOH (50 mL) was added 2N NaOH_{(aq)} (1.5 mL, 3.00 mmol). After addition, the reaction mixture was stirred for overnight.

The reaction was monitored by TLC. After reaction was completed, the solvent was removed under reduced pressure. The residue was diluted with ice-cold water and acidified with 1N HCl. The precipitated solid was collected by filtration to afford the desired product Compound **69c** ((E)-3-(4-(((5-oxo-5,6,7,8-tetrahydro-naphthalen-2-yl)oxy)methyl)phenyl)acrylic acid) (0.46 g, 1.43 mmol, yield 99%).

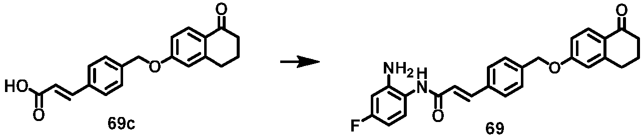

To a solution of Compound **69c** ((E)-3-(4-(((5-oxo-5,6,7,8-tetrahydro-naphthalen-2-yl)oxy)methyl)phenyl)acrylic acid) (0.07 g, 0.22 mmol), 4-fluoro-1,2-phenylenediamine (0.03 g, 0.26 mmol) and DMAP (0.01 g, 0.11 mmol) in THF (30 mL) was added NMM (0.03 g, 0.33 mmol) and EDCI (0.06 g, 0.33 mmol) at 0 °C. After addition, the reaction mixture was warmed to room temperature and stirred for 6 hours.

The reaction was monitored by TLC. After reaction was completed, the solvent was removed under reduced pressure. The residue was diluted with *sat.* NH₄Cl_{(aq)} and extracted with EtOAc. The organic phase was dried with MgSO₄ and concentrated under reduced pressure. The crude product was purified by silica gel column chromatography using ethyl acetate/hexane = 1/1 as elution to yield the desired product Compound **69** ((E)-N-(2-amino-4-fluorophenyl)-3-(4-(((5-oxo-5,6,7,8-tetra-hydronaphthalen-2-yl)oxy)methyl)p henyl)acrylamide) (0.03 g, 0.07 mmol, yield 31%).

Compound **69,** ¹H-NMR (500 MHz, CD₃OD): δ 7.94-7.93(d, 1H), 7.69-7.64(m, 3H), 7.52-7.50(d, 2H), 7.17-7.14(m, 1H), 6.97-6.95(dd, 1H), 6.92(s, 1H), 6.87-6.83(d, 1H), 6.58-6.56(dd, 1H), 6.43-6.39(td, 1H), 5.22(s, 2H), 2.97-2.95(t, 2H), 2.61-2.58(t, 2H), 2.12-2.09(m, 2H). ESI-MS *m*/*z* calcd for C₂₆H₂₃FN₂O₃ 430.17, found 431 [M+H]⁺.

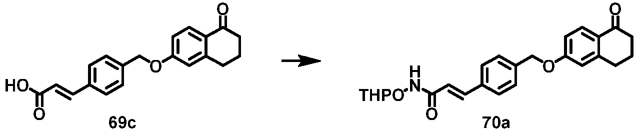

To a solution of Compound **69c** ((E)-3-(4-(((5-oxo-5,6,7,8-tetrahydro-naphthalen-2-yl)oxy)methyl)phenyl)acrylic acid) (0.12 g, 0.37 mmol), NH₂OTHP (0.07 g, 0.56 mmol) and DMAP (0.02 g, 0.19 mmol) in CH₂Cl₂ (20 mL) was added NMM (0.06 g, 0.56 mmol) and EDCI (0.11 g, 0.56 mmol) at 0 °C. After addition, the reaction mixture was warmed to room temperature and stirred for overnight.

The reaction was monitored by TLC. After reaction was completed, the solvent was removed under reduced pressure. The residue was diluted with ice-cold water and extracted with EtOAc. The organic phase was dried with MgSO₄ and concentrated under reduced pressure. The crude product was purified by silica gel column chromatography using ethyl acetate/hexane = 1/1 as elution to yield the desired product Compound **70a** ((E)-3-(4-(((5-oxo-5,6,7,8-tetrahydronaphthalen-2-yl)oxy)-methyl)phenyl)-N-((tetrahydro-2H-py ran-2-yl)oxy)acrylamide) (0.09 g, 0.22 mmol, yield 60%).

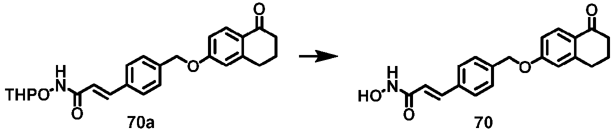

To a solution of Compound **70a** ((E)-3-(4-(((5-oxo-5,6,7,8-tetrahydro-naphthalen-2-yl)oxy)-methyl)phenyl)-N-((tetrahydro-2H-p yran-2-yl)oxy)acrylamide) (0.09 g, 0.22 mmol) in CH₂Cl₂ (20 mL) was added 2N HCl (excess, in diethyl ether). After addition, the reaction mixture was stirred for overnight.

The reaction was monitored by TLC. After reaction was completed, the solvent was removed under reduced pressure. The residue was diluted with diethyl ether, the precipitated solid was collected by filtration to afford the desired product Compound **70** ((E)-N-hydroxy-3-(4-(((5-oxo-5,6,7,8-tetrahydronaphthalen-2-yl)oxy)-methyl)phenyl)acrylamide ) (0.05 g, 0.16 mmol, yield 71%).

Compound **70,** ¹H-NMR (500 MHz, CD₃OD): δ 7.94-7.92(d, 1H), 7.67-7.63(m, 1H), 7.59-7.57(m, 2H), 7.49-7.48(d, 2H), 6.96-6.93(dd, 1H), 6.91(s, 1H), 6.50-6.47(d, 1H), 5.20(s, 2H), 2.97-2.94(t, 2H), 2.60-2.58(t, 2H), 2.11-2.09(m, 2H). ESI-MS *m*/*z* calcd for C₂₀H₁₉NO₄ 337.13, found 338 [M+H]⁺.

### Synthesis of Compounds 71~72

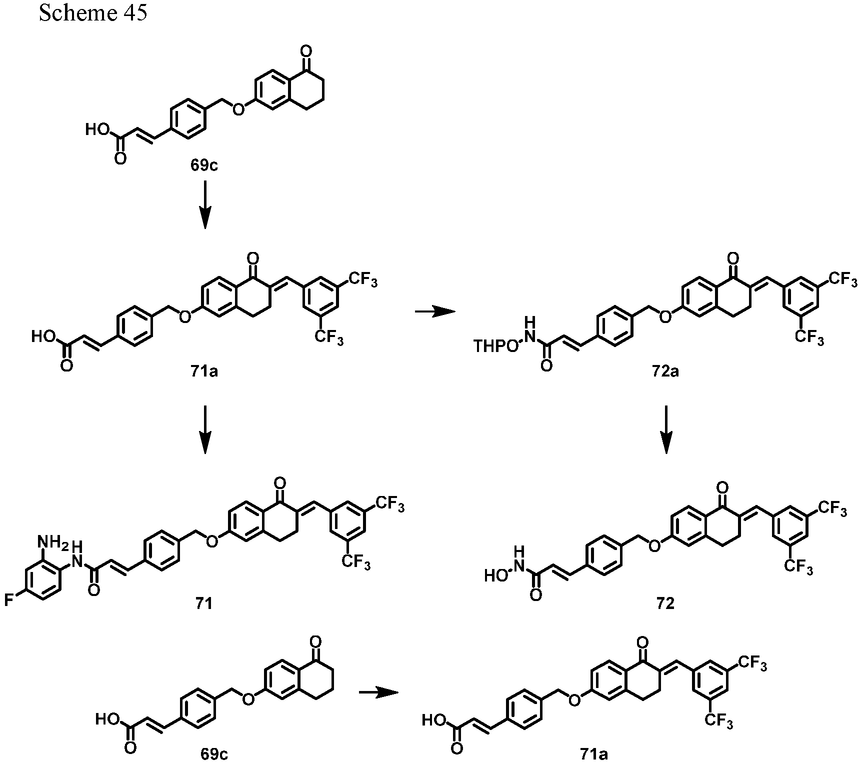

To a solution of Compound **69c** ((E)-3-(4-(((5-oxo-5,6,7,8-tetra-hydronaphthalen-2-yl)oxy)methyl)phenyl)acrylic acid) (0.47 g, 1.45 mmol) and 3,5-Bis(trifluoromethyl)benzaldehyde (0.42 g, 1.74 mmol) in MeOH (30 mL) was added 2N NaOH (1.50 mL, 3.00 mmol). After addition, the reaction mixture was stirred for overnight.

After reaction was completed, the solvent was removed under reduced pressure. The residual was diluted with ice-cold water and acidified with 1N HCl. The precipitated solid was collected by filtration and washed with 30% MeOH/water to get Compound **71a** ((E)-3-(4-(((6-((E)-3,5-bis(trifluoromethyl)benzylidene)-5-oxo-5,6,7,8-tetrahydronaphthalen-2-yl )oxy)methyl)phenyl)acrylic acid) (0.56 g, 1.03 mmol , yield 71%).

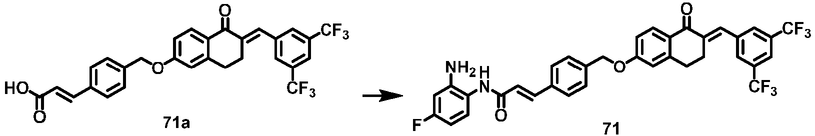

To a solution of Compound **71a** ((E)-3-(4-(((6-((E)-3,5-bis(trifluoro-methyl)benzylidene)-5-oxo-5,6,7,8-tetrahydronaphthalen-2-yl)oxy)methyl)phenyl)-acrylic acid) (0.20 g, 0.37 mmol), 4-fluoro-1,2-phenylenediamine (0.06 g, 0.44 mmol) and DMAP (0.02 g, 0.19 mmol) in THF (30 mL) was added NMM (0.06 g, 0.55 mmol) and EDCI (0.11 g, 0.55 mmol) at 0 °C. After addition, the reaction mixture was warmed to room temperature and stirred for 6 hours.

The reaction was monitored by TLC. After reaction was completed, the solvent was removed under reduced pressure. The residue was diluted with *sat.* NH₄Cl_{(aq)} and extracted with EtOAc. The organic phase was dried with MgSO₄ and concentrated under reduced pressure. The crude product was purified by silica gel column chromatography using ethyl acetate/hexane = 1/1 as elution to yield the desired product Compound 71 ((E)-N-(2-amino-4-fluorophenyl)-3-(4-(((6-((E)-3,5-bis(tri-fluoromethyl)benzylidene)-5-oxo-5,6, 7,8-tetrahydronaphthalen-2-yl)oxy)methyl)-phenyl)acrylamide) (0.06 g, 0.10 mmol, yield 26%).

Compound **71,** ¹H-NMR (500 MHz, CDCl₃): δ 8.15-8.13(d, 1H), 7.85-7.82(m, 4H), 7.78-7.75(d, 1H), 7.60-7.58(d, 2H), 7.48-7.46(d, 2H), 7.18(s, 1H), 6.99-6.97(d, 1H), 6.81(s, 1H), 6.62-6.59(d, 1H), 6.54-6.52(m, 2H), 5.17(s, 2H), 4.04(s, 2H), 3.06-2.96(m, 4H). ESI-MS *m*/*z* calcd for C₃₅H₂₅F₇N₂O₃ 654.18, found 655 [M+H]⁺.

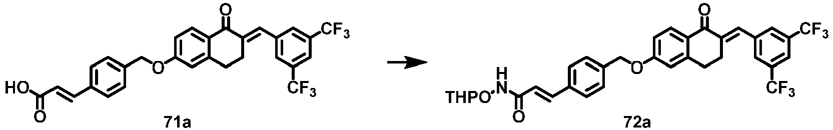

To a solution of Compound **71a** ((E)-3-(4-(((6-((E)-3,5-bis(trifluoro-methyl)benzylidene)-5-oxo-5,6,7,8-tetrahydronaphthalen-2-yl)oxy)methyl)phenyl)-acrylic acid) (0.20 g, 0.37 mmol), NH₂OTHP (0.06 g, 0.55 mmol) and DMAP (0.02 g, 0.19 mmol) in CH₂Cl₂ (20 mL) was added NMM (0.06 g, 0.55 mmol) and EDCI (0.11 g, 0.55 mmol) at 0 °C. After addition, the reaction mixture was warmed to room temperature and stirred for overnight.

The reaction was monitored by TLC. After reaction was completed, the solvent was removed under reduced pressure. The residue was diluted with ice-cold water and extracted with EtOAc. The organic phase was dried with MgSO₄ and concentrated under reduced pressure. The crude product was purified by silica gel column chromatography using ethyl acetate/hexane = 2/1 as elution to yield the desired product Compound **72a** ((E)-3-(4-(((6-((E)-3,5-bis(trifluoromethyl)benzylidene)-5-oxo-5,6,7,8-tetrahydronaphthalen-2-yl )oxy)methyl)phenyl)-N-((tetrahydro-2H-pyran-2-yl)oxy)acrylamide) (0.12 g, 0.19 mmol, yield 50%).

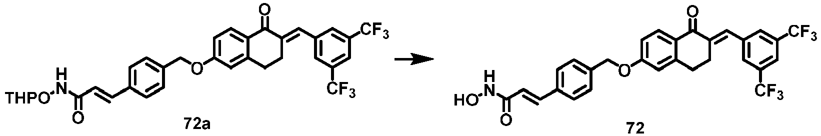

To a solution of Compound **72a** ((E)-3-(4-(((6-((E)-3,5-bis(trifluoro-methyl)benzylidene)-5-oxo-5,6,7,8-tetrahydronaphthalen-2-yl)oxy)methyl)phenyl)-N-((tetrahydro-2H-pyran-2-yl)oxy)acrylamide) (0.12 g, 0.19 mmol) in CH₂Cl₂ (20 mL) was added 2N HCl (excess, in diethyl ether). After addition, the reaction mixture was stirred for overnight.

The reaction was monitored by TLC. After reaction was completed, the solvent was removed under reduced pressure. The residue was diluted with diethyl ether, the precipitated solid was collected by filtration to afford the desired product Compound **72** ((E)-3-(4-(((6-((E)-3,5-bis(trifluoromethyl)benzylidene)-5-oxo-5,6,7,8-tetrahydronaphthalen-2-yl )oxy)methyl)phenyl)-N-hydroxyacrylamide) (0.08 g, 0.14 mmol, yield 74%).

Compound **72,** ¹H-NMR (500 MHz, CDCl₃): δ 8.14-8.12(d, 1H), 7.85-7.82(m, 4H), 7.74-7.71(d, 1H), 7.56-7.54(d, 2H), 7.46-7.44(d, 2H), 6.98-6.96(d, 1H), 6.80(s, 1H), 6.38(br, 1H), 5.16(s, 2H), 3.05-2.95(m, 4H). ESI-MS *m*/*z* calcd for C₂₉H₂₁F₆NO₄ 561.14, found 562 [M+H]⁺.

### Synthesis of Compound 73

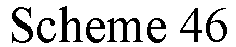

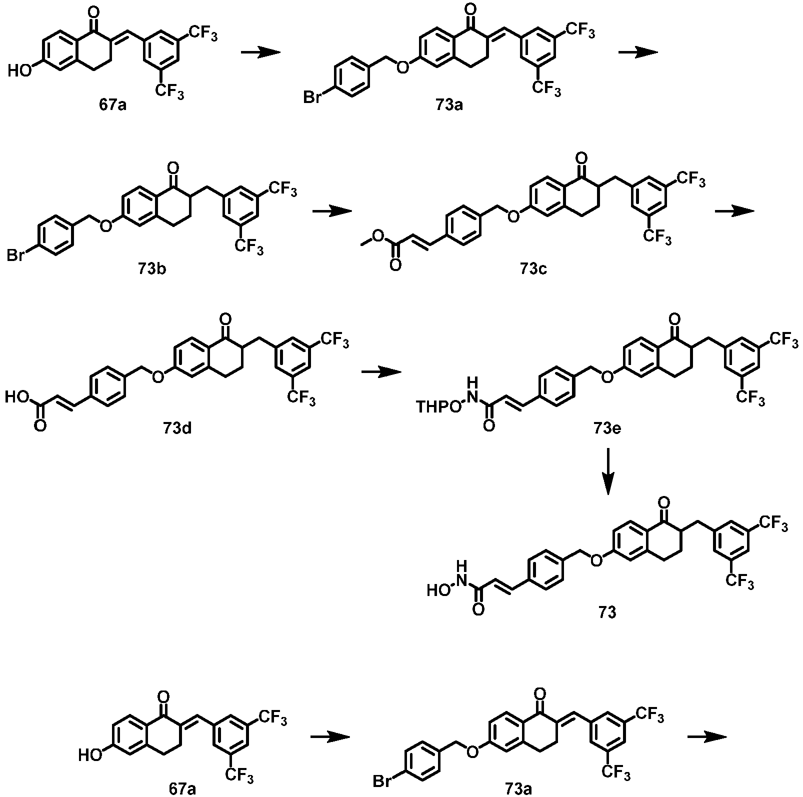

To a solution of Compound **67a** ((E)-2-(3,5-bis(trifluoromethyl)-benzylidene)-6-hydroxy-3,4-dihydronaphthalen-1(2H)-one) (386 mg, 1.00 mmol) in ACN (10 mL) at RT in pressure tube was added 4-Bromobenzyl bromide (250 mg, 1.00 mmol), Potassium carbonate (138 mg, 1.00 mmol). The tube was sealed and stirred at 95 °C for 3 hours.

After cooling to RT, removed the solvent in vacuo, the crude product was purified by column chromatography (EtOAc:*n*-hexane = 1:10) to afford the product Compound **73a** ((E)-2-(3,5-bis(trifluoromethyl)benzylidene)-6-((4-bromobenzyl)oxy)-3,4-dihydronaphthalen-1( 2H)-one) (400 mg, 72%).

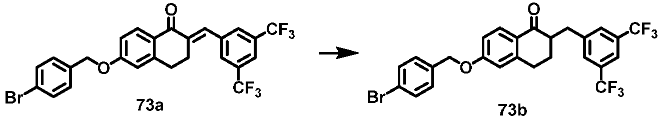

To a solution of Compound **73a** ((E)-2-(3,5-bis(trifluoromethyl)-benzylidene)-6-((4-bromobenzyl)oxy)-3,4-dihydronaphthalen-1( 2H)-one) (400 mg, 0.72 mmol) in DMF (30 mL) at RT was added NH₄Cl (386 mg, 7.21 mmol), Zinc (239 mg, 3.60 mmol).

The mixture was sealed and stirred at RT for 12 hours, then diluted with EtOAc and washed with water followed brine. The combined organic layer was dried over anhydrous MgSO₄. After remove the solvent, purification by column chromatography (EtOAc:*n*-hexane = 1:10) to afford the product Compound **73b** (2-(3,5-bis(trifluoromethyl)benzyl)-6-((4-bromobenzyl)oxy)-3,4-dihydronaphthalen-1(2H)-one) (317 mg, 79%).

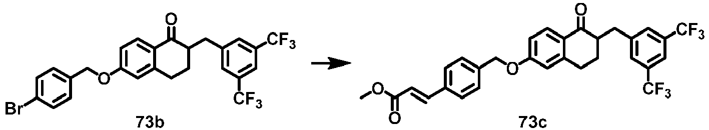

To a solution of Compound **73b** (2-(3,5-bis(trifluoromethyl)benzyl)-6-((4-bromobenzyl)oxy)-3,4-dihydronaphthalen-1(2H)-one) (317 mg, 0.57 mmol) in ACN (8 mL) was added mthyl acrylate (1.3 mL, 14.2 mmol) at RT in pressure tube, followed Et₃N (8 mL), PPh₃ (222 mg, 0.85 mmol). The mixture was stirred at RT for 5 mins then degas with nitrogen, followed added Pd(OAc)₂ (76 mg, 0.34 mmol) and seal the tube. The reaction was stirred at 100 °C for 21 hours.

After cooling to RT, removed the solvent in vacuo and purified with column chromatography (EtOAc:*n*-hexane = 1:5 ) to afford the product Compound **73c** (methyl (E)-3-(4-(((6-(3,5-bis(trifluoromethyl)benzyl)-5-oxo-5,6,7,8-tetrahydronaphthalen-2-yl)oxy)met hyl)phenyl)acrylate) (266 mg, 83%).

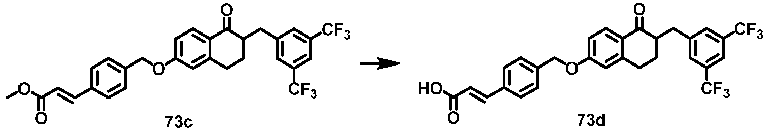

To a solution of Compound **73c** (methyl (E)-3-(4-(((6-(3,5-bis(trifluoro-methyl)benzyl)-5-oxo-5,6,7,8-tetrahydronaphthalen-2-yl)oxy)met hyl)phenyl)acrylate) (253 mg, 0.45 mmol) in co-solvent MeOH (20 mL) and DCM (40 mL) was added NaOH aqueous solution (4.0 M, 3 mL) at RT.

The mixture was stirred at RT for 12 hours, then removed the solvent in vacuo followed quench with HCl aqueous solution (1.0 M) to pH = 4. The mixture was extracted with EtOAc, then take the organic layer washed with brine. The combined organic layer was dried over anhydrous MgSO₄. After remove the solvent, purified with column chromatography (EtOAc:*n*-hexane = 1:2) to afford the product. Compound **73d** ((E)-3-(4-(((6-(3,5-bis(trifluoromethyl)benzyl)-5-oxo-5,6,7,8-tetra-hydronaphthalen-2-yl)oxy)me thyl)phenyl)acrylic acid) (76 mg, 31%).

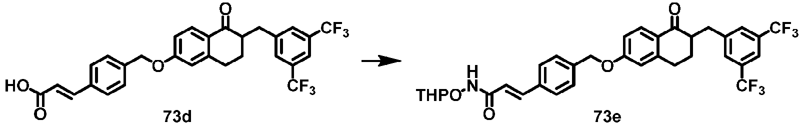

To a solution of Compound **73d** ((E)-3-(4-(((6-(3,5-bis(trifluoro-methyl)benzyl)-5-oxo-5,6,7,8-tetra-hydronaphthalen-2-yl)oxy)m ethyl)phenyl)acrylic acid) (98 mg, 0.18 mmol) in DMF (30 mL) at 0 °C under nitrogen was added EDC hydrochloride (40 mg, 0.21 mmol), followed DMAP (27 mg, 0.21 mmol ) and *O*-(Tetrahydro-2H-pyran-2-yl)hydroxylamine (25 mg, 0.21 mmol). The mixture was allowed to warm to RT and stirred for 20 hours.

After removing the solvent, the crude product was purified by column chromatography (EtOAc:*n*-hexane = 1:1) to afford the solid Compound **73e** ((E)-3-(4-(((6-(3,5-bis(trifluoromethyl)benzyl)-5-oxo-5,6,7,8-tetrahydronaphthalen-2-yl)oxy)-me thyl)phenyl)-N-((tetrahydro-2H-pyran-2-yl)oxy)acrylamide) (67 mg, 58%).

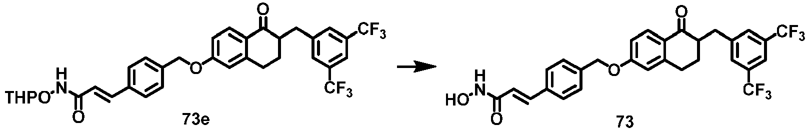

To a solution of Compound **73e** ((E)-3-(4-(((6-(3,5-bis(trifluoromethyl)-benzyl)-5-oxo-5,6,7,8-tetrahydronaphthalen-2-yl)oxy)-m ethyl)phenyl)-N-((tetrahydro-2H-pyran-2-yl)oxy)acrylamide) (29 mg, 0.04 mmol) in DCM (10 mL) at RT was added hydrogen chloride solution 2.0 M in diethyl ether (0.1 mL, 0.20 mmol).

The reaction was stirred at RT for 3 hours, then the solid was filtered out to afford the product Compound **73** ((E)-3-(4-(((6-(3,5-bis(trifluoromethyl)benzyl)-5-oxo-5,6,7,8-tetrahydronaphthalen-2-yl)oxy)met hyl)phenyl)-N-hydroxyacrylamide) (15 mg, 60%).

Compound **73,** ¹H-NMR (500 MHz, d₆-DMSO): δ 7.99 (s, 2H), 7.91 (s, 1H), 7.86-7.84 (d, 1H), 7.59-7.58 (d, 2H), 7.48-7.44 (d, 3H), 6.98-6.97 (d, 1H), 6.94 (s, 1H), 6.50-6.47 (d, 1H), 5.21 (s,2H), 3.43-3.40 (m, 1H), 3.00-2.86 (m, 4H), 1.98-1.94 (m, 1H), 1.73-1.70 (m, 1H). ESI-MS *m*/*z* calcd for C₂₉H₂₃F₆NO₂ 563.15, found 564 [M+H]⁺.

### Synthesis of Compound 74

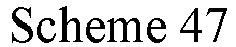

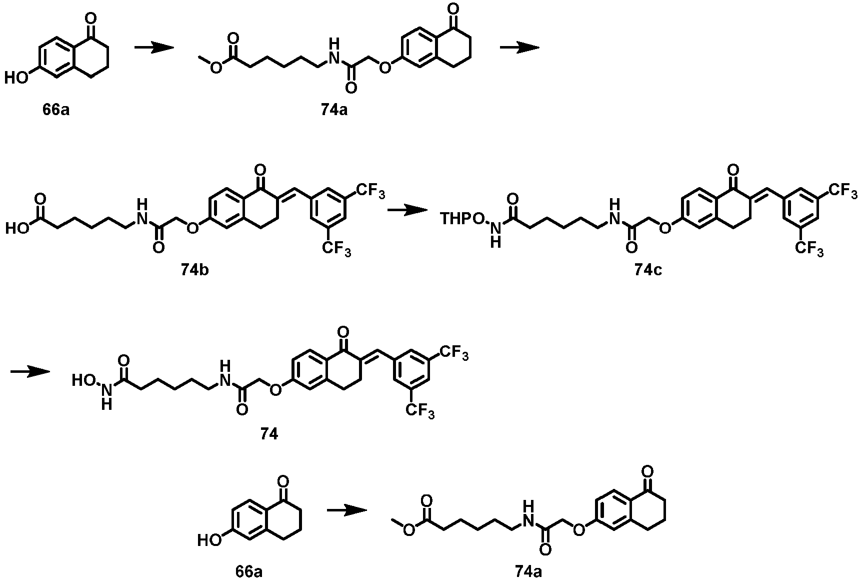

To a solution of Compound **66a** (6-Hydroxy-3,4-dihydro-1(2H)-naphthalenone) (0.12 g, 0.74 mmol) and K₂CO₃ (0.31 g, 2.22 mmol) in acetone (20 mL) was added methyl 6-(2-chloroacetamido)hexanoate (0.25 g, 1.11 mmol) and KI (catalytic amount). After addition, the reaction mixture was refluxed for overnight.

The reaction was monitored by TLC. After reaction was completed, the solvent was removed under reduced pressure. The residue was diluted with *sat.* NH₄Cl_{(aq)} and extracted with EtOAc. The organic phase was dried with MgSO₄ and concentrated under reduced pressure. The crude product was purified by silica gel column chromatography using ethyl acetate/hexane = 2/1 as elution to yield the desired product Compound **74a** (methyl 6-(2-((5-oxo-5,6,7,8-tetrahydronaphthalen-2-yl)oxy)-acetamido)hexanoate) (0.24 g, 0.69 mmol, yield 93%).

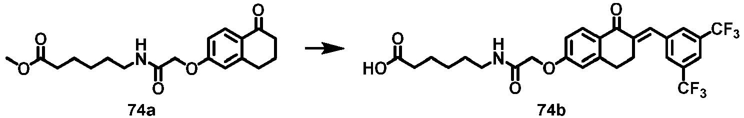

To a solution of Compound **74a** (methyl 6-(2-((5-oxo-5,6,7,8-tetrahydronaphthalen-2-yl)oxy)-acetamido)hexanoate) (0.42 g, 1.21 mmol) and 3,5-Bis(trifluoromethyl)benzaldehyde (0.29 g, 1.21 mmol) in MeOH (50 mL) was added 2N NaOH_{(aq)} (1.5 mL, 3.00 mmol). After addition, the reaction mixture was stirred for overnight.

The reaction was monitored by TLC. After reaction was completed, the solvent was removed under reduced pressure. The residue was diluted with ice-cold water and acidified with 1N HCl. The precipitated solid was collected by filtration to afford the desired product Compound **74b** ((E)-6-(2-((6-(3,5-bis(trifluoromethyl)-benzylidene)-5-oxo-5,6,7,8-tetrahydronaphthalen-2-yl)ox y)acetamido)hexanoic acid) (0.45 g, 0.81 mmol, yield 67%).

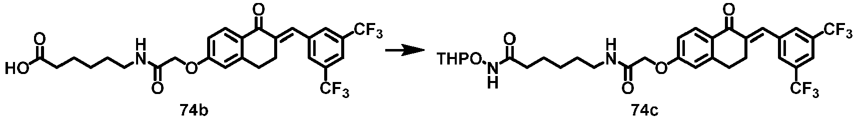

To a solution of Compound **74b** ((E)-6-(2-((6-(3,5-bis(trifluoromethyl)-benzylidene)-5-oxo-5,6,7,8-tetrahydronaphthalen-2-yl)ox y)acetamido)hexanoic acid) (0.45 g, 0.81 mmol), NH₂OTHP (0.12 g, 1.06 mmol) and DMAP (0.05 g, 0.41 mmol) in CH₂Cl₂ (50 mL) was added NMM (0.12 g, 1.22 mmol) and EDCI (0.20 g, 1.06 mmol) at 0 °C. After addition, the reaction mixture was warmed to room temperature and stirred for overnight.

The reaction was monitored by TLC. After reaction was completed, the solvent was removed under reduced pressure. The residue was diluted with ice-cold water and extracted with EtOAc. The organic phase was dried with MgSO₄ and concentrated under reduced pressure. The crude product was purified by silica gel column chromatography using ethyl acetate/hexane = 2/1 as elution to yield the desired product Compound **74c** ((E)-6-(2-((6-(3,5-bis(trifluoromethyl)benzylidene)-5-oxo-5,6,7,8-tetrahydronaphthalen-2-yl)oxy )acetamido)-N-((tetrahydro-2H-pyran-2-yl)oxy)hexanamide) (0.24 g, 0.36 mmol, yield 45%).

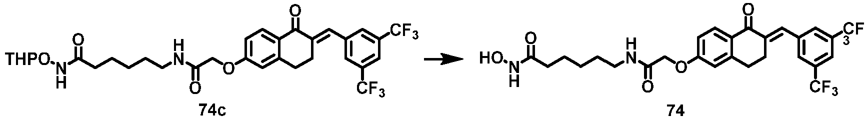

To a solution of Compound **74c** ((E)-6-(2-((6-(3,5-bis(trifluoromethyl)-benzylidene)-5-oxo-5,6,7,8-tetrahydronaphthalen-2-yl)ox y)acetamido)-N-((tetrahydro-2H-pyran-2-yl)oxy)hexanamide) (0.24 g, 0.36 mmol) in CH₂Cl₂ (30 mL) was added 2N HCl (excess, in diethyl ether). After addition, the reaction mixture was stirred for overnight.

The reaction was monitored by TLC. After reaction was completed, the solvent was removed under reduced pressure. The residue was diluted with diethyl ether, the precipitated solid was collected by filtration to afford the desired product Compound **74** ((E)-6-(2-((6-(3,5-bis(trifluoromethyl)benzylidene)-5-oxo-5,6,7,8-tetra-hydronaphthalen-2-yl)ox y)acetamido)-N-hydroxyhexanamide) (0.02 g, 0.03 mmol, yield 10%).

Compound **74,** ¹H-NMR (500 MHz, CD₃OD): δ 8.07-8.06(d, 1H), 8.02(s, 2H), 7.97(s, 1H), 7.82(s, 1H), 7.05-7.03(d, 1H), 6.92(s, 1H), 4.62(s, 2H), 3.31-3.27(m, 2H), 3.09-3.00(m, 4H), 2.09-2.06(t, 2H), 1.64-1.54(m, 4H), 1.35-1.33(m, 2H). ESI-MS *m*/*z* calcd for C₂₇H₂₆F₆N₂O₅ 572.17, found 573 [M+H]⁺.

### Synthesis of Compound 75

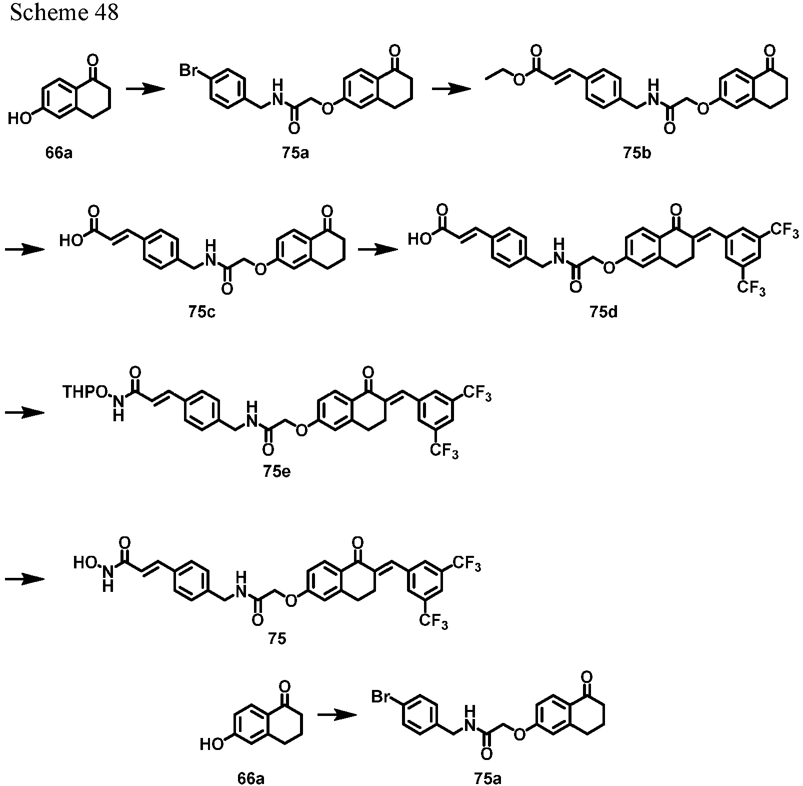

To a solution of Compound **66a** (6-Hydroxy-3,4-dihydro-1(2H)-naphthalenone) (0.50 g, 3.08 mmol) and K₂CO₃ (0.85 g, 6.17 mmol) in acetone (50 mL) was added N-(4-bromobenzyl)-2-chloroacetamide (0.81 g, 3.08 mmol) and KI (catalytic amount). After addition, the reaction mixture was refluxed for overnight.

The reaction was monitored by TLC. After reaction was completed, the solvent was removed under reduced pressure. The residue was diluted with *sat.* NH₄Cl_{(aq)} and extracted with EtOAc. The organic phase was dried with MgSO₄ and concentrated under reduced pressure. The crude product was purified by silica gel column chromatography using ethyl acetate/hexane = 1/1 as elution to yield the desired product Compound **75a** (N-(4-bromobenzyl)-2-((5-oxo-5,6,7,8-tetrahydronaphthalen-2-yl)oxy)acetamide) (0.97 g, 2.49 mmol, yield 81%).

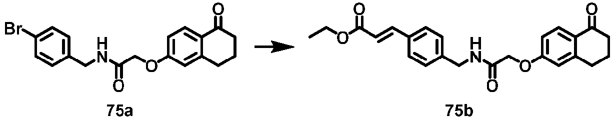

To a solution of Compound **75a** (N-(4-bromobenzyl)-2-((5-oxo-5,6,7,8-tetrahydronaphthalen-2-yl)oxy)acetamide) (0.97 g, 2.49 mmol), triphenylphosphine (0.26 g, 1.00 mmol), ethyl acrylate (0.32 g, 3.24 mmol) in DMF/TEA (50 mL, 1:1) was added Pd(OAc)₂ (0.03 g, 0.12 mmol). After addition, the reaction mixture was heated to 100 °C and stirred for overnight.

The reaction was monitored by TLC. After reaction was completed, the reaction mixture was diluted with *sat.* NH₄Cl_{(aq)} and extracted with EtOAc. The organic phase was dried with MgSO₄ and concentrated under reduced pressure. The crude product was purified by silica gel column chromatography using ethyl acetate/hexane = 1/1 as elution to yield the desired product Compound **75b** (ethyl (E)-3-(4-((2-((5-oxo-5,6,7,8-tetrahydronaphthalen-2-yl)oxy)acetamido)methyl)phenyl)acrylate) (0.62 g, 1.51 mmol, yield 61%).

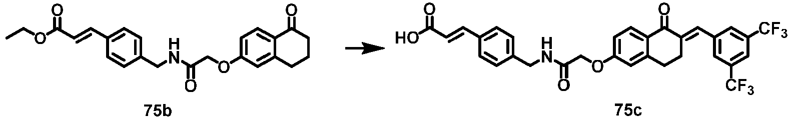

To a solution of Compound **75b** (ethyl (E)-3-(4-((2-((5-oxo-5,6,7,8-tetrahydronaphthalen-2-yl)oxy)acetamido)methyl)phenyl)acrylate) (0.62 g, 1.51 mmol) and 3,5-Bis(trifluoromethyl)benzaldehyde (0.40 g, 1.66 mmol) in MeOH (50 mL) was added 2N NaOH_{(aq)} (1.5 mL, 3.00 mmol). After addition, the reaction mixture was stirred for overnight.

The reaction was monitored by TLC. After reaction was completed, the solvent was removed under reduced pressure. The residue was diluted with ice-cold water and acidified with 1N HCl. The precipitated solid was collected by filtration to afford the desired product Compound **75c** ((E)-3-(4-((2-((6-((E)-3,5-bis(trifluoro-methyl)benzylidene)-5-oxo-5,6,7,8-tetrahydronaphthalen-2-yl)oxy)acetamido)-methyl)phenyl)acrylic acid) (0.89 g, 1.47 mmol, yield 97%).

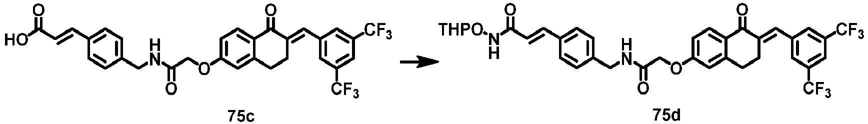

To a solution of Compound **75c** ((E)-3-(4-((2-((6-((E)-3,5-bis(trifluoro-methyl)benzylidene)-5-oxo-5,6,7,8-tetrahydronaphthalen-2-yl)oxy)acetamido)-methyl)phenyl)acrylic acid) (0.89 g, 1.47 mmol), NH₂OTHP (0.22 g, 1.91 mmol) and DMAP (0.09 g, 0.74 mmol) in CH₂Cl₂ (50 mL) was added NMM (0.22 g, 2.21 mmol) and EDCI (0.42 g, 2.21 mmol) at 0 °C. After addition, the reaction mixture was warmed to room temperature and stirred for overnight.

The reaction was monitored by TLC. After reaction was completed, the solvent was removed under reduced pressure. The residue was diluted with ice-cold water and extracted with EtOAc. The organic phase was dried with MgSO₄ and concentrated under reduced pressure. The crude product was purified by silica gel column chromatography using ethyl acetate/hexane = 2/1 as elution to yield the desired product Compound **75d** ((E)-3-(4-((2-((6-((E)-3,5-bis(trifluoromethyl)benzylidene)-5-oxo-5,6,7,8-tetrahydronaphthalen-2-yl)oxy)acetamido)methyl)phenyl)-N-((tetrahydro-2H-pyran-2-yl)oxy)acrylamide) (0.49 g, 0.69 mmol, yield 47%).

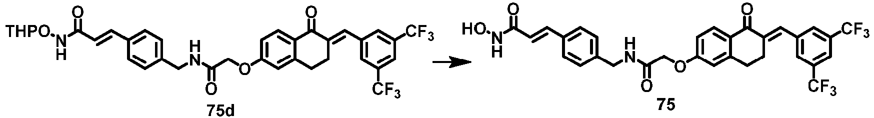

To a solution of Compound **75d** ((E)-3-(4-((2-((6-((E)-3,5-bis(trifluoro-methyl)benzylidene)-5-oxo-5,6,7,8-tetrahydronaphthalen-2-yl)oxy)acetamido)-methyl)phenyl)-N-((tetrahydro-2H-pyran-2-yl)oxy)acrylamide) (0.49 g, 0.69 mmol) in CH₂Cl₂ (50 mL) was added 2N HCl (excess, in diethyl ether). After addition, the reaction mixture was stirred for overnight.

The reaction was monitored by TLC. After reaction was completed, the solvent was removed under reduced pressure. The residue was diluted with diethyl ether, the precipitated solid was collected by filtration to afford the desired product Compound **75** ((E)-3-(4-((2-((6-((E)-3,5-bis(trifluoromethyl)benzylidene)-5-oxo-5,6,7,8-tetrahydronaphthalen-2-yl)oxy)acetamido)methyl)phenyl)-N-hydroxyacrylamide) (0.11 g, 0.18 mmol, yield 26%).

Compound **75,** ¹H-NMR (500 MHz, CD₃OD): δ 8.79-8.76(t, 1H), 8.07-8.05(d, 1H), 8.02(s, 2H), 7.97(s, 1H), 7.82(s, 1H), 7.56-7.52(d, 1H), 7.50-7.49(d, 2H), 7.32-7.30(d, 2H), 7.04-7.03(d, 1H), 6.92(s, 1H), 6.45-6.42(d, 1H), 4.70(s, 2H), 4.48(s, 2H), 3.09-3.06(t, 2H), 2.98-2.96(t, 2H). ESI-MS *m*/*z* calcd for C₃₁H₂₄F₆N₂O₅ 618.16, found 619 [M+H]⁺.

### Synthesis of Compounds 78~79

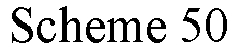

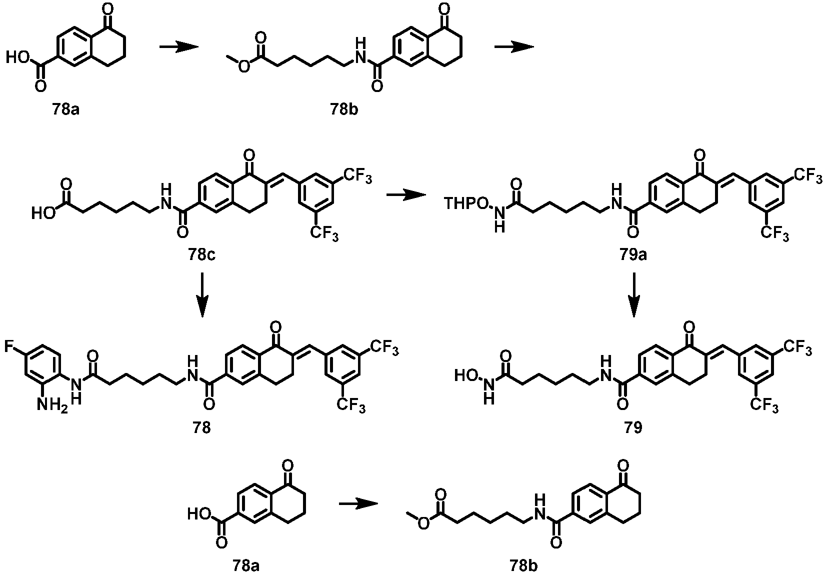

To a solution of Compound **78a** (5-Oxo-5,6,7,8-tetrahydro-2-naphthalene-carboxylic acid) (1.00 g, 5.26 mmol), methyl 6-aminohexanoate hydrogen chloride (1.15 g, 6.31 mmol) and DMAP (0.32 g, 2.63 mmol) in DCM (100 mL) was added DIPEA (2.04 g, 15.77 mmol) and EDCI (1.21 g, 6.31 mmol) at 0°C. After addition, the reaction mixture was slowly warmed to RT and stirred for overnight.

After reaction was completed, the solvent was removed under reduced pressure. The residual was diluted with EtOAc and washed with *Sat.* NH₄Cl. The combined organic layers were washed with brine and dried over MgSO₄ and concentrated in vacuo to give the desired product Compound **78b** (methyl 6-(5-oxo-5,6,7,8-tetrahydro-naphthalene-2-carboxamido)hexanoate) (1.61 g, 5.07 mmol, yield 96%).

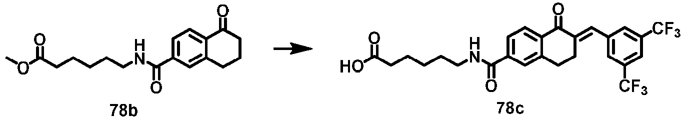

To a solution of Compound **78b** (methyl 6-(5-oxo-5,6,7,8-tetrahydro-naphthalene-2-carboxamido)hexanoate) (1.61 g, 5.07 mmol) in MeOH (50 mL) was added 2N NaOH (5.00 mL, 10.00 mmol). After addition, the reaction mixture was stirred for overnight.

After reaction was completed, the solvent was removed under reduced pressure. The residual was diluted with ice-cold water and acidified with 1N HCl. The precipitated solid was collected by filtration and washed with 30% MeOH/water to give the intermedia product. It was then treated with 3,5-Bis(trifluoromethyl)-benzaldehyde (1.44 g, 5.93 mmol) in MeOH (50 mL) was added 2N NaOH (5.00 mL, 10.00 mmol). After addition, the reaction mixture was stirred for overnight.

After reaction was completed, the solvent was removed under reduced pressure. The residual was diluted with ice-cold water and acidified with 1N HCl. The precipitated solid was collected by filtration and washed with 30% MeOH/water to get Compound **78c** ((E)-6-(6-(3,5-bis(trifluoromethyl)benzylidene)-5-oxo-5,6,7,8-tetrahydro-naphthalene-2-carboxa mido)hexanoic acid) (2.44 g, 4.63 mmol , yield 94%).

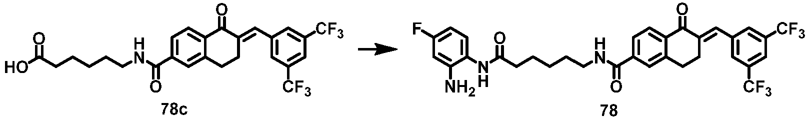

To a solution of Compound **78c** ((E)-6-(6-(3,5-bis(trifluoromethyl)-benzylidene)-5-oxo-5,6,7,8-tetrahydro-naphthalene-2-carbox amido) hexanoic acid) (1.48 g, 2.80 mmol), 4-fluoro-1,2-phenylenediamine (0.35 g, 2.80 mmol) and DMAP (0.34 g, 2.80 mmol) in THF (100 mL) was added NMM (0.28 g, 2.80 mmol) and EDCI (0.64 g, 3.36 mmol) at 0 °C. After addition, the reaction mixture was warmed to room temperature and stirred for 6 hours.

The reaction was monitored by TLC. After reaction was completed, the solvent was removed under reduced pressure. The residue was diluted with *sat.* NH₄Cl_{(aq)} and extracted with EtOAc. The organic phase was dried with MgSO₄ and concentrated under reduced pressure. The crude product was purified by silica gel column chromatography using ethyl acetate/hexane = 2/1 as elution to yield the desired product Compound **78** ((E)-N-(6-((2-amino-4-fluorophenyl)amino)-6-oxohexyl)-6-(3,5-bis(trifluoromethyl)-benzyliden e)-5-oxo-5,6,7,8-tetrahydronaphthalene-2-carboxamide) (0.05 g, 0.08 mmol, yield 3%).

Compound **78,** ¹H-NMR (500 MHz, CD₃OD): δ 8.13-8.11(d, 1H), 8.05(s, 2H), 7.99(s, 1H), 7.88(s, 1H), 7.80-7.76(m, 2H), 7.01-6.98(m, 1H), 6.53-6.51(m, 1H), 6.35-6.31(m, 1H), 3.45-3.42(t, 2H), 3.12-3.03(m, 4H), 2.45-2.42(t, 2H), 1.80-1.68(m, 4H), 1.52-1.50(m, 2H). ESI-MS *m*/*z* calcd for C₃₂H₂₈F₇N₃O₃ 635.20, found 636 [M+H]⁺.

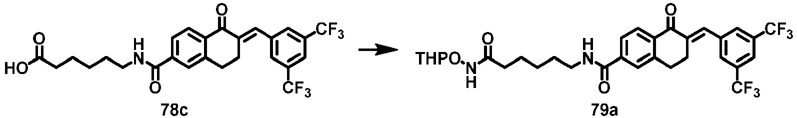

To a solution of Compound **78c** ((E)-6-(6-(3,5-bis(trifluoromethyl)-benzylidene)-5-oxo-5,6,7,8-tetrahydro-naphthalene-2-carbox amido) hexanoic acid) (0.45 g, 0.85 mmol), NH₂OTHP (0.15 g, 1.28 mmol) and DMAP (0.05 g, 0.43 mmol) in CH₂Cl₂ (50 mL) was added NMM (0.13 g, 1.28 mmol) and EDCI (0.25 g, 1.28 mmol) at 0 °C. After addition, the reaction mixture was warmed to room temperature and stirred for overnight.

The reaction was monitored by TLC. After reaction was completed, the solvent was removed under reduced pressure. The residue was diluted with ice-cold water and extracted with EtOAc. The organic phase was dried with MgSO₄ and concentrated under reduced pressure. The crude product was purified by silica gel column chromatography using ethyl acetate/hexane = 2/1 as elution to yield the desired product Compound **79a** ((E)-6-(3,5-bis(trifluoromethyl)benzylidene)-5-oxo-N-(6-oxo-6-(((tetrahydro-2H-pyran-2-yl)-ox y)amino)hexyl)-5,6,7,8-tetrahydronaphthalene-2-carboxamide) (0.17 g, 0.27 mmol, yield 32%).

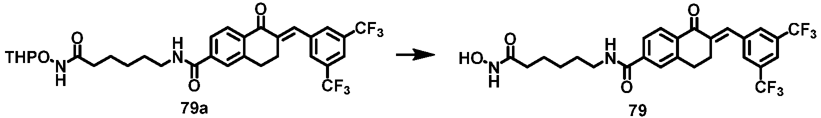

To a solution of Compound **79a** ((E)-6-(3,5-bis(trifluoromethyl)benzylidene)-5-oxo-N-(6-oxo-6-(((tetrahydro-2H-pyran-2-yl)-ox y)amino)hexyl)-5,6,7,8-tetrahydronaphthalene-2-carboxamide) (0.17 g, 0.27 mmol) in CH₂Cl₂ (20 mL) was added 2N HCl (excess, in diethyl ether). After addition, the reaction mixture was stirred for overnight.

The reaction was monitored by TLC. After reaction was completed, the solvent was removed under reduced pressure. The residue was diluted with diethyl ether, the precipitated solid was collected by filtration to afford the desired product Compound **79** ((E)-6-(3,5-bis(trifluoromethyl)benzylidene)-N-(6-(hydroxyamino)-6-oxohexyl)-5-oxo-5,6,7,8-te trahydronaphthalene-2-carboxamide) (0.04 g, 0.07 mmol, yield 27%).

Compound **79,** ¹H-NMR (500 MHz, CD₃OD): δ 8.14-8.12(d, 1H), 8.05(s, 2H), 7.99(s, 1H), 7.88(s, 1H), 7.80-7.78(m, 2H), 3.40-3.38(t, 2H), 3.13-3.07(m, 4H), 2.13-2.10(t, 2H), 1.70-1.62(m, 4H), 1.45-1.40(m, 2H). ESI-MS *m*/*z* calcd for C₂₆H₂₄F₆N₂O₄ 542.16, found 543 [M+H]⁺.

### Synthesis of Compounds 80~81

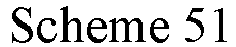

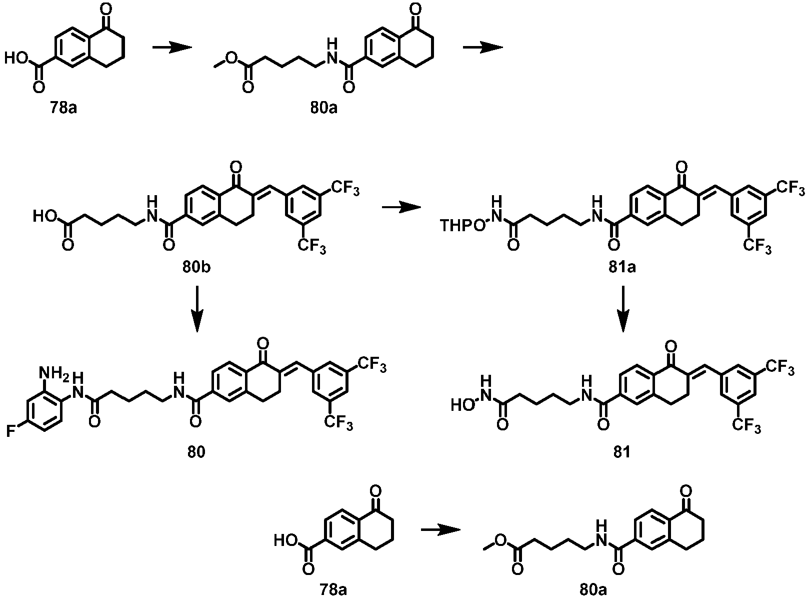

To a solution of Compound **78a** (5-Oxo-5,6,7,8-tetrahydro-2-naphthalene-carboxylic acid) (1.00 g, 5.26 mmol), methyl 5-aminopentanoate hydrogen chloride (0.97 g, 5.79 mmol) and DMAP (0.32 g, 2.63 mmol) in DCM (100 mL) was added DIPEA (2.04 g, 15.77 mmol) and EDCI (1.21 g, 6.31 mmol) at 0°C. After addition, the reaction mixture was slowly warmed to RT and stirred for overnight.

After reaction was completed, the solvent was removed under reduced pressure. The residual was diluted with EtOAc and washed with *Sat.* NH₄Cl. The combined organic layers were washed with brine and dried over MgSO₄ and concentrated in vacuo to give the desired product Compound **80a** (methyl 5-(5-oxo-5,6,7,8-tetrahydro-naphthalene-2-carboxamido)pentanoate) (1.37 g, 4.53 mmol, yield 86%).

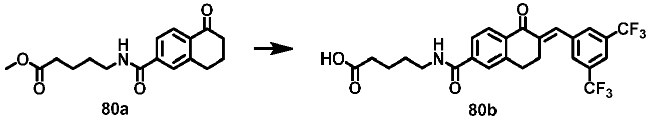

To a solution of Compound **80a** (methyl 5-(5-oxo-5,6,7,8-tetrahydro-naphthalene-2-carboxamido)pentanoate) (1.37 g, 4.53 mmol) and 3,5-Bis-(trifluoromethyl)benzaldehyde (1.27 g, 5.25 mmol) in MeOH (50 mL) was added 2N NaOH (5.00 mL, 10.00 mmol). After addition, the reaction mixture was stirred for overnight.

After reaction was completed, the solvent was removed under reduced pressure. The residual was diluted with ice-cold water and acidified with 1N HCl. The precipitated solid was collected by filtration and washed with 30% MeOH/water to get Compound **80b** ((E)-5-(6-(3,5-bis(trifluoromethyl)benzylidene)-5-oxo-5,6,7,8-tetrahydro-naphthalene-2-carboxa mido)pentanoic acid) (1.72 g, 3.35 mmol , yield 74%).

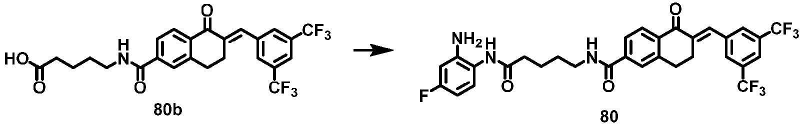

To a solution of Compound **80b** ((E)-5-(6-(3,5-bis(trifluoromethyl)-benzylidene)-5-oxo-5,6,7,8-tetrahydro-naphthalene-2-carbox amido)pentanoic acid) (0.20 g, 0.39 mmol), 4-fluoro-1,2-phenylenediamine (0.05 g, 0.39 mmol) and DMAP (0.05 g, 0.39 mmol) in THF (30 mL) was added NMM (0.04 g, 0.39 mmol) and EDCI (0.10 g, 0.51 mmol) at 0 °C. After addition, the reaction mixture was warmed to room temperature and stirred for 6 hours.

The reaction was monitored by TLC. After reaction was completed, the solvent was removed under reduced pressure. The residue was diluted with *sat.* NH₄Cl_{(aq)} and extracted with EtOAc. The organic phase was dried with MgSO₄ and concentrated under reduced pressure. The crude product was purified by silica gel column chromatography using ethyl acetate/hexane = 2/1 as elution to yield the desired product Compound **80** ((E)-N-(5-((2-amino-4-fluorophenyl)amino)-5-oxopentyl)-6-(3,5-bis(trifluoromethyl)-benzyliden e)-5-oxo-5,6,7,8-tetrahydronaphthalene-2-carboxamide) (0.10 g, 0.16 mmol, yield 42%).

Compound **80,** ¹H-NMR (500 MHz, CD₃OD): δ 9.02(s, 1H), 8.68-8.66(t, 1H), 8.21(s, 2H), 8.14(s, 1H), 8.05-8.04(d, 1H), 7.85-7.84(m, 3H), 7.11-7.08(t, 1H), 6.49-6.46(dd, 1H), 6.31-6.27(td, 1H), 5.13(s, 2H), 3.09-3.01(m, 4H), 2.36-2.33(t, 2H), 1.66-1.57(m, 4H). ESI-MS *m*/*z* calcd for C₃₁H₂₆F₇N₃O₃ 621.19, found 622 [M+H]⁺.

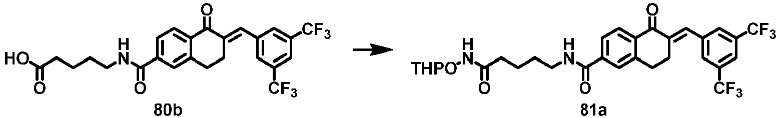

To a solution of Compound **80b** ((E)-5-(6-(3,5-bis(trifluoromethyl)-benzylidene)-5-oxo-5,6,7,8-tetrahydro-naphthalene-2-carbox amido)pentanoic acid) (0.20 g, 0.39 mmol), NH₂OTHP (0.06 g, 0.51 mmol) and DMAP (0.02 g, 0.20 mmol) in CH₂Cl₂ (30 mL) was added NMM (0.06 g, 0.59 mmol) and EDCI (0.11 g, 0.59 mmol) at 0 °C. After addition, the reaction mixture was warmed to room temperature and stirred for overnight.

The reaction was monitored by TLC. After reaction was completed, the solvent was removed under reduced pressure. The residue was diluted with ice-cold water and extracted with EtOAc. The organic phase was dried with MgSO₄ and concentrated under reduced pressure. The crude product was purified by silica gel column chromatography using ethyl acetate/hexane = 2/1 as elution to yield the desired product Compound **81a** ((E)-6-(3,5-bis(trifluoromethyl)benzylidene)-5-oxo-N-(5-oxo-5-(((tetrahydro-2H-pyran-2-yl)oxy )amino)pentyl)-5,6,7,8-tetrahydronaphthalene-2-carboxamide) (0.11 g, 0.18 mmol, yield 47%).

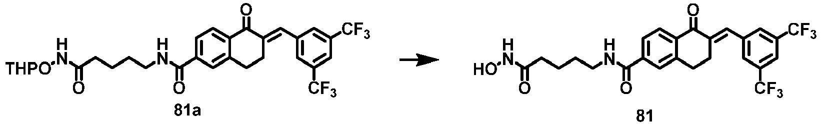

To a solution of Compound **81a** ((E)-6-(3,5-bis(trifluoromethyl)benzylidene)-5-oxo-N-(5-oxo-5-(((tetrahydro-2H-pyran-2-yl)oxy )amino)pentyl)-5,6,7,8-tetrahydro-naphthalene-2-carboxamide) (0.11 g, 0.18 mmol) in CH₂Cl₂ (20 mL) was added 2N HCl (excess, in diethyl ether). After addition, the reaction mixture was stirred for overnight.

The reaction was monitored by TLC. After reaction was completed, the solvent was removed under reduced pressure. The residue was diluted with diethyl ether, the precipitated solid was collected by filtration to afford the desired product Compound **81** ((E)-6-(3,5-bis(trifluoromethyl)benzylidene)-N-(5-(hydroxyamino)-5-oxopentyl)-5-oxo-5,6,7,8-t etrahydronaphthalene-2-carboxamide) (0.01 g, 0.02 mmol, yield 13%).

Compound **81,** ¹H-NMR (500 MHz, CD₃OD): δ 8.14-8.12(d, 1H), 8.05(s, 2H), 7.99(s, 1H), 7.88(s, 1H), 7.80-7.78(m, 2H), 3.43-3.40(t, 2H), 3.15-3.12(t, 2H), 3.08-3.06(t, 2H), 2.17-2.14(t, 2H), 1.73-1.64(m, 4H). ESI-MS *m*/*z* calcd for C₂₅H₂₂F₆N₂O₄ 528.15, found 529 [M+H]⁺.

### Synthesis of Compounds 82~83

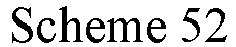

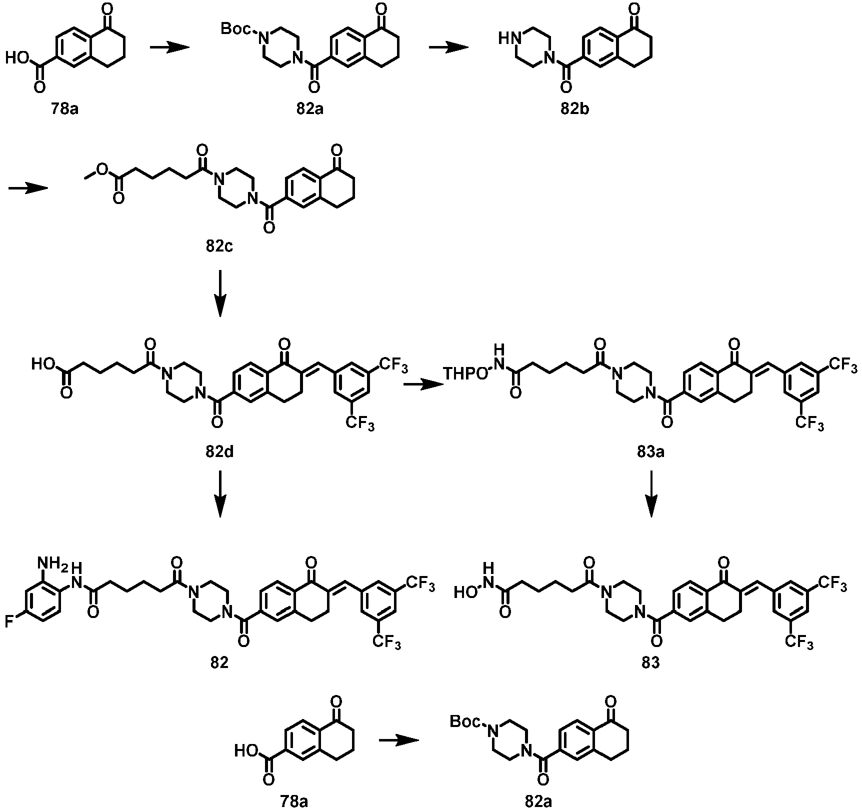

To a solution of Compound **78a** (5-Oxo-5,6,7,8-tetrahydro-2-naphthalene-carboxylic acid) (0.50 g, 2.63 mmol), 1-Boc-piperazine (0.54 g, 2.89 mmol) and DMAP (0.16 g, 1.31 mmol) in DCM (50 mL) was added DIPEA (0.51 g, 3.94 mmol) and EDCI (0.66 g, 3.42 mmol) at 0°C. After addition, the reaction mixture was slowly warmed to RT and stirred for overnight.

After reaction was completed, the solvent was removed under reduced pressure. The residual was diluted with EtOAc and washed with *Sat.* NH₄Cl. The combined organic layers were washed with brine and dried over MgSO₄ and concentrated in vacuo to give the desired product Compound **82a** (tert-butyl 4-(5-oxo-5,6,7,8-tetrahydro-naphthalene-2-carbonyl)piperazine-1-carboxylate) (0.98 g, 2.73 mmol, yield 94%).

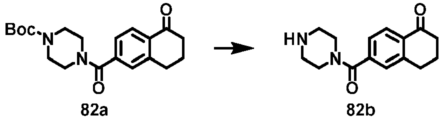

To a solution of Compound **82a** (tert-butyl 4-(5-oxo-5,6,7,8-tetrahydro-naphthalene-2-carbonyl)piperazine-1-carboxylate) (0.98 g, 2.73 mmol) in CH₂Cl₂ (20 mL) was added 2N HCl (excess, in diethyl ether). After addition, the reaction mixture was stirred for overnight.

The reaction was monitored by TLC. After reaction was completed, the solvent was removed under reduced pressure to afford the intermediate Compound **82b** (6-(piperazine-1-carbonyl)-3,4-dihydronaphthalen-1(2H)-one hydrogen chloride).

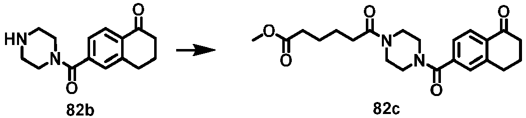

And then, to the solution of Compound **82b** (6-(piperazine-1-carbonyl)-3,4-dihydronaphthalen-1(2H)-one hydrogen chloride), 6-methoxy-6-oxohexanoic acid (0.57 g, 3.54 mmol) and DMAP (0.43 g, 3.54 mmol) in CH₂Cl₂ (30 mL) was added DIPEA (1.06 g, 8.18 mmol) and EDCI (0.78 g, 4.09 mmol) at 0°C. After addition, the reaction mixture was slowly warmed to RT and stirred for overnight.

The reaction was monitored by TLC. After reaction was completed, the solvent was removed under reduced pressure. The residual was diluted with EtOAc and washed with *Sat.* NH₄Cl. The combined organic layers were washed with brine and dried over MgSO₄ and concentrated in vacuo to give the desired product Compound **82c** (methyl 6-oxo-6-(4-(5-oxo-5,6,7,8-tetrahydronaphthalene-2-carbonyl)piperazin-1-yl)hexanoate) (1.01 g, 2.52 mmol, yield 92%).

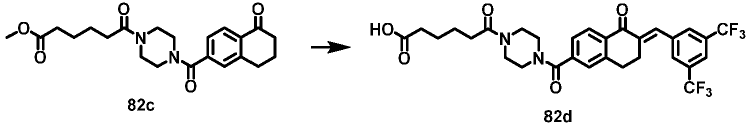

To a solution of Compound **82c** (methyl 6-oxo-6-(4-(5-oxo-5,6,7,8-tetrahydronaphthalene-2-carbonyl)piperazin-1-yl)hexanoate) (1.01 g, 2.52 mmol) and 3,5-Bis(trifluoromethyl)benzaldehyde (0.61 g, 2.52 mmol) in MeOH (50 mL) was added 2N NaOH (5.00 mL, 10.00 mmol). After addition, the reaction mixture was stirred for overnight.

After reaction was completed, the solvent was removed under reduced pressure. The residual was diluted with ice-cold water and acidified with 1N HCl. The precipitated solid was collected by filtration and washed with 30% MeOH/water to get Compound **82d** ((E)-6-(4-(6-(3,5-bis(trifluoromethyl)benzylidene)-5-oxo-5,6,7,8-tetra-hydronaphthalene-2-carbo nyl)piperazin-1-yl)-6-oxohexanoic acid) (0.95 g, 1.56 mmol , yield 62%).

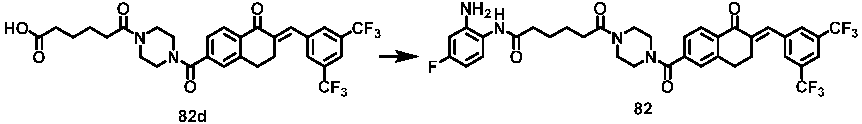

To a solution of Compound **82d** ((E)-6-(4-(6-(3,5-bis(trifluoromethyl)-benzylidene)-5-oxo-5,6,7,8-tetra-hydronaphthalene-2-carb onyl)-piperazin-1-yl)-6-oxohexanoic acid ) (0.23 g, 0.38 mmol), 4-fluoro-1,2-phenylenediamine (0.05 g, 0.38 mmol) and DMAP (0.05 g, 0.38 mmol) in THF (30 mL) was added NMM (0.04 g, 0.38 mmol) and EDCI (0.10 g, 0.49 mmol) at 0 °C. After addition, the reaction mixture was warmed to room temperature and stirred for 6 hours.

The reaction was monitored by TLC. After reaction was completed, the solvent was removed under reduced pressure. The residue was diluted with *sat.* NH₄Cl_{(aq)} and extracted with EtOAc. The organic phase was dried with MgSO₄ and concentrated under reduced pressure. The crude product was purified by silica gel column chromatography using ethyl acetate/methanol = 10/1 as elution to yield the desired product Compound **82** ((E)-N-(2-amino-4-fluorophenyl)-6-(4-(6-(3,5-bis(trifluoromethyl)benzylidene)-5-oxo-5,6,7,8-tet rahydronaphthalene-2-carbonyl)piperazin-1-yl)-6-oxohexanamide) (0.06 g, 0.08 mmol, yield 21%).

Compound **82,** ¹H-NMR (500 MHz, CD₃OD): δ 8.16-8.14(d, 1H), 8.05(s, 2H), 7.99(s, 1H), 7.89(s, 1H), 7.46-7.44(d, 1H), 7.42(s, 1H), 7.05(m, 1H), 6.54-6.52(m, 1H), 6.39-6.35(m, 1H), 3.81-3.45(m, 8H), 3.15-3.12(t, 2H), 3.07-3.04(t, 2H), 2.52-2.44(m, 4H),1.81-1.74(m, 2H), 1.33-1.29(m, 2H). ESI-MS *m*/*z* calcd for C₃₆H₃₃F₇N₄O₄ 718.24, found 719 [M+H]⁺.

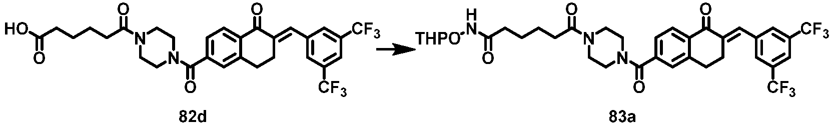

To a solution of Compound **82d** ((E)-6-(4-(6-(3,5-bis(trifluoromethyl)-benzylidene)-5-oxo-5,6,7,8-tetra-hydronaphthalene-2-carb onyl)-piperazin-1-yl)-6-oxohexanoic acid ) (0.37 g, 0.60 mmol), NH₂OTHP (0.09 g, 0.79 mmol) and DMAP (0.04 g, 0.30 mmol) in CH₂Cl₂ (50 mL) was added NMM (0.09 g, 0.91 mmol) and EDCI (0.17 g, 0.91 mmol) at 0 °C. After addition, the reaction mixture was warmed to room temperature and stirred for overnight.

The reaction was monitored by TLC. After reaction was completed, the solvent was removed under reduced pressure. The residue was diluted with ice-cold water and extracted with EtOAc. The organic phase was dried with MgSO₄ and concentrated under reduced pressure. The crude product was purified by silica gel column chromatography using ethyl acetate/methanol = 10/1 as elution to yield the desired product Compound **83a** ((E)-6-(4-(6-(3,5-bis(trifluoromethyl)benzylidene)-5-oxo-5,6,7,8-tetrahydronaphthalene-2-carbo nyl)piperazin-1-yl)-6-oxo-N-((tetrahydro-2H-pyran-2-yl)oxy)hexanamide)
(0.23 g, 0.32 mmol, yield 53%).

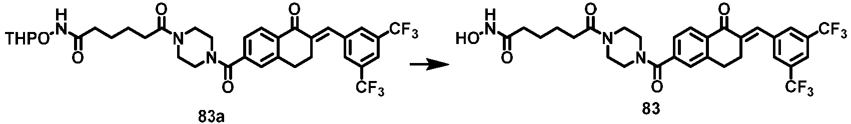

To a solution of Compound **83a** ((E)-6-(4-(6-(3,5-bis(trifluoromethyl)-benzylidene)-5-oxo-5,6,7,8-tetrahydronaphthalene-2-carbo nyl)piperazin-1-yl)-6-oxo-N-((tetrahydro-2H-pyran-2-yl)oxy)hexanamide) (0.23 g, 0.32 mmol) in CH₂Cl₂ (20 mL) was added 2N HCl (excess, in diethyl ether). After addition, the reaction mixture was stirred for overnight.

The reaction was monitored by TLC. After reaction was completed, the solvent was removed under reduced pressure. The residue was diluted with diethyl ether, the precipitated solid was collected by filtration to afford the desired product Compound **83** ((E)-6-(4-(6-(3,5-bis(trifluoromethyl)benzylidene)-5-oxo-5,6,7,8-tetrahydro-naphthalene-2-carbo nyl)piperazin-1-yl)-N-hydroxy-6-oxohexanamide) (0.09 g, 0.15 mmol, yield 47%).

Compound **83,** ¹H-NMR (500 MHz, CD₃OD): δ 8.16-8.15(d, 1H), 8.05(s, 2H), 7.99(s, 1H), 7.89(s, 1H), 7.47-7.46(d, 1H), 7.43(s, 1H), 3.81-3.45(m, 8H), 3.15-3.13(t, 2H), 3.07-3.05(t, 2H), 2.48-2.43(m, 2H), 2.15-2.12(m, 2H), 1.65-1.53(m, 2H), 1.34-1.29(m, 2H). ESI-MS *m*/*z* calcd for C₃₀H₂₉F₆N₃O₅ 625.20, found 626 [M+H]⁺.

### Example II: Autotaxin / HDAC Inhibition Assay

To prove that the Compounds of the present disclosure have the effect of inhibiting the activity of Autotaxin and HDAC (dual ATX/HDAC inhibitors), the following experiments were performed for evaluation.

### Autotaxin Inhibition Screening Assay

ATX (autotaxin) activity was measured by choline release from LPC in presence or absence of test compounds (Compounds at concentrations of 4µM, 20µM and 50µM). Twenty (20) ng ATX (10803, Cayman, MI, USA) was incubated with 100 µM 14:0 LPC (855575P, Avanti, AL, USA) in a final volume of 100 µL buffer containing 50 mM Tris pH 8.0, 0.01% Triton X-100, 50 mM CaCl₂, 1 unit/ml choline oxidase, 2 unit/ml horseradish peroxidase (HRP), 2 mM homovanilic acid (HVA). The relative amount of released choline was measured by HVA fluorescence in a 96-well plate. Fluorescent intensity was determined at λex/λem = 320/450 nm every 60 sec for 90 min with a SpectraMax i3 (Molecular Devices, CA, USA). Data analysis was performed using GraphPad Prism (GraphPad, La Jolla CA, USA).$Inhibition % = \left[1-\left({Slope}_{TA}-{Slope}_{Blank}\right)/\left({Slope}_{Vehicle}-{Slope}_{Blank}\right)\right] \times 100 %$

### HDAC Inhibition Screening Assay

The HDAC1/ HDAC2/ HDAC3 *in vitro* activities are detected by Enzo Life Sciences' HDAC1/ HDAC2/ HDAC3 Fluorimetric Drug Discovery Kit. The assay is based on the FLUOR DE LYS^{®} Substrate and FLUOR DE LYS^{®} Developer combination. The assay procedure has two steps. First, the FLUOR DE LYS^{®} Substrate, which comprises an acetylated lysine side chain, is incubated with HDAC1/ HDAC2/ HDAC3, respectively. Deacetylation of the substrate sensitizes the substrate so that, in the second step, treatment with the FLUOR DE LYS^{®}Developer produces a fluorophore. The signals of the test compounds (Compounds 1~83; at concentrations of 2µM, 10µM and 50µM) in 96-well plates are detected at 360 nm or 485 nm excitation and 460 nm or 530 nm emission with a Molecular Devices SpectraMax i3 Imaging Cytometer. The HDAC1/ HDAC2/ HDAC3 activities are expressed in percentage of basal control minus baseline control.

The inhibition rate of the Compounds on the activity of autotaxin and HDAC are summarized in Table 1 below.

**Table 1**

| Compound # | HDAC | | | | | | | | | Autotaxin | | |
|---|---|---|---|---|---|---|---|---|---|---|---|---|
| | HDAC1 | | | HDAC2 | | | HDAC3 | | | LPC-CR | | |
| | 2 (µM) | 10 (µM) | 50 (µM) | 2 (µM) | 10 (µM) | 50 (µM) | 2 (µM) | 10 (µM) | 50 (µM) | 4 (µM) | 20 (µM) | 50 (µM) |
| 1 | A | A | A | A | A | A | A | A | A | C | C | C |
| 4 | B | A | A | B | A | A | C | B | A | A | A | A |
| 5 | C | C | C | C | C | C | C | C | C | B | B | B |
| 6 | C | C | C | C | C | C | C | C | C | B | B | B |
| 7 | C | C | B | C | C | B | C | C | B | C | B | B |
| 8 | B | A | A | A | A | A | C | A | A | A | A | A |
| 9 | C | C | C | C | C | C | C | C | C | C | C | C |
| 10 | C | B | A | C | A | A | C | B | A | A | A | A |
| 11 | C | C | C | C | C | C | C | C | C | A | A | A |
| 12 | C | B | A | B | A | A | B | A | A | A | A | A |
| 13 | C | C | C | C | C | C | C | C | C | B | B | B |
| 14 | C | B | A | C | B | A | C | C | A | A | A | A |
| 66 | C | C | C | C | C | C | C | C | C | C | C | C |
| 67 | C | C | C | C | C | C | C | C | C | C | C | C |
| 69 | C | C | C | C | C | B | C | C | C | C | C | C |
| 70 | B | A | A | A | A | A | A | A | A | C | B | B |
| 71 | C | C | C | C | C | C | C | C | C | C | C | C |
| 72 | C | C | B | C | C | B | C | C | B | B | B | B |
| 73 | C | C | C | C | C | C | C | C | C | C | C | C |
| 74 | C | A | A | B | A | A | C | A | A | A | A | A |
| 75 | C | C | B | C | C | B | C | C | B | B | B | B |
| 78 | C | C | C | C | C | C | C | C | C | A | A | A |
| 79 | B | A | A | B | A | A | B | A | A | A | A | A |
| 80 | C | C | C | C | C | C | C | C | C | C | C | B |
| 81 | C | C | B | C | C | B | C | B | B | A | A | A |
| 82 | C | C | C | C | C | C | C | C | B | A | A | A |
| 83 | C | C | B | C | C | B | C | C | B | A | A | A |

| | | | | | | | | | | | | |
|---|---|---|---|---|---|---|---|---|---|---|---|---|
| A = >80% inhibition; B = 50~80% inhibition; C = <50% inhibition. | | | | | | | | | | | | |

The experimental results revealed that the Compounds of the present disclosure have inhibition activities for ATX (autotaxin) and HDAC (histone deacetylase) and are useful as dual inhibitors of HDAC and autotaxin.

## Claims

1. A compound of Formula (I-1) or a geometric isomer, enantiomer, diastereomer, racemate, pharmaceutically acceptable salt or solvate thereof: 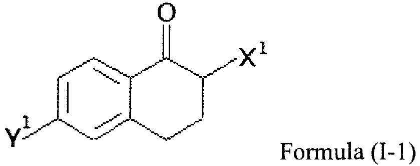 wherein,
X¹ is hydrogen, or R¹-A¹, and R¹ is a single bond or a double bond, A¹ is an aryl optionally substituted with -CF₃ groups;
Y¹ is -NHC(O)R²C(O)NHR³, -R²C(O)NHR³, -R²C(O)NHR⁴C(O)NHR³, -C(O)NHR²C(O)NHR³, -OCH₂R⁵C(O)NHR³, -OCH₂R⁵R²C(O)NHR³, or -C(O)R⁶C(O)R ²C(O)NHR³, wherein R² and R⁴ are independently selected from a group consisting of C₁-C₁₀ alkyl, C₁-C₁₀ alkenyl, and C₁-C₁₀ alkoxy, R³ is independently selected from a group consisting of -OH, an aryl optionally substituted with at least one of a halogen, an alkyl, an alkoxy or -NH₂, and when R³ is an aryl substituted with -NH₂, any hydrogen in the NH₂ may be optionally substituted with alkyl, R⁵ is an aryl and R⁶ is a heterocyclic alkyl,
wherein,
when Y¹ is OCH₂R⁵C(O)NHR³, R³ is an aryl optionally substituted with at least one of a halogen, an alkyl, an alkoxy or -NH₂.

2. The compound according to claim 1, wherein the compound of Formula (I-1) is selected from the group consisting of the compounds delineated in Table A or a geometric isomer, enantiomer, diastereomer, racemate, pharmaceutically acceptable salt or solvate thereof.
**Table A:**
| (No.) | Structure | (No.) | Structure |
|---|---|---|---|
| 1 | 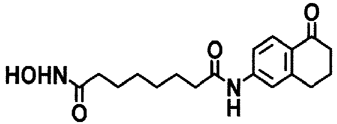 | | |
| 4 | 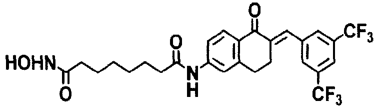 | 69 | 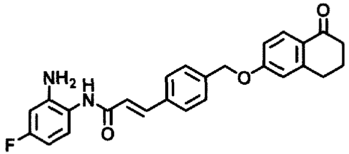 |
| 5 | 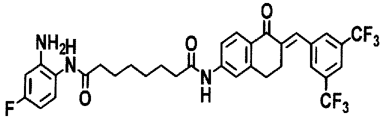 | 70 | 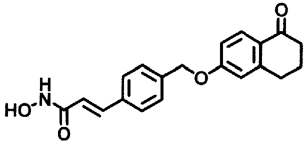 |
| 6 | 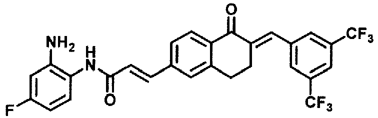 | 71 | 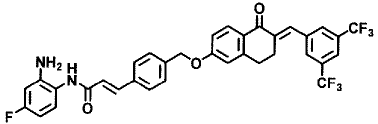 |
| 7 | 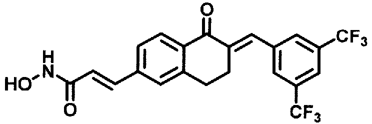 | 72 | 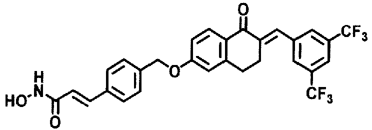 |
| 8 | 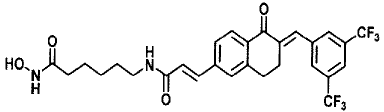 | 73 | 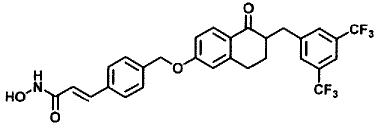 |
| 9 | 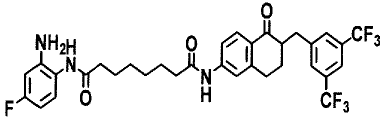 | 74 | 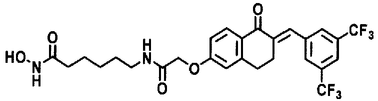 |
| 10 | 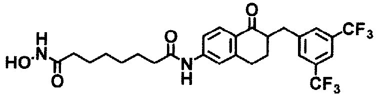 | 75 | 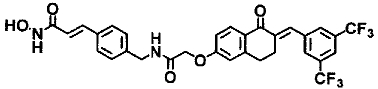 |
| 11 | 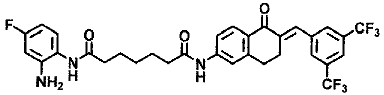 | 78 | 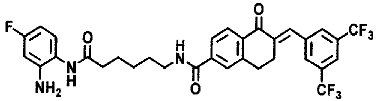 |
| 12 | 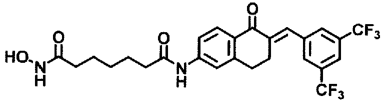 | 79 | 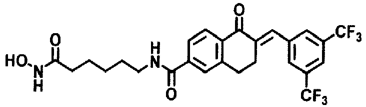 |
| 13 | 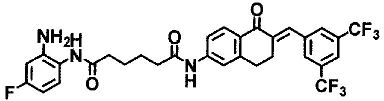 | 80 | 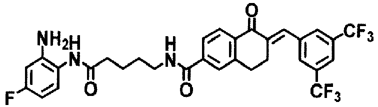 |
| 14 | 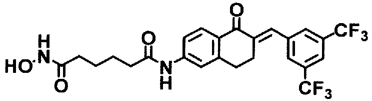 | 81 | 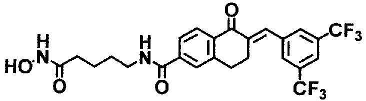 |
| 66 | 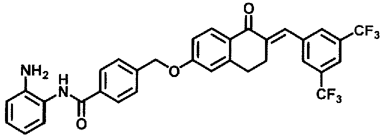 | 82 | 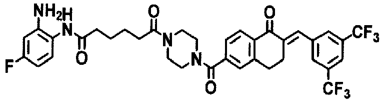 |
| 67 | 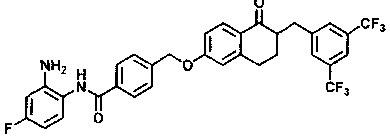 | 83 | 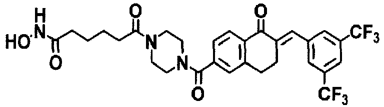 |

3. The compound according to claim 1 or 2, wherein the compound is a dual autotaxin / HDAC inhibitor.

4. A pharmaceutical composition comprising a therapeutically effective amount of the compound according to claim 1 or 2 and a pharmaceutically acceptable carrier.

## Patentansprüche

1. Verbindung der Formel (I-1) oder ein geometrisches Isomer, Enantiomer, Diastereomer, Racemat, pharmazeutisch verträgliches Salz oder Solvat davon: 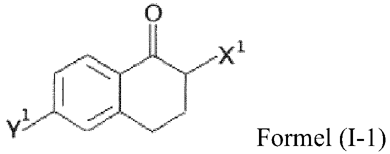 wobei
X¹ Wasserstoff oder R¹-A¹ ist und R¹ eine Einfachbindung oder eine Doppelbindung ist, A¹ ein gegebenenfalls mit -CF₃-Gruppen substituiertes Aryl ist;
Y¹ -NHC(O)R²C(O)NHR³, -R²C(O)NHR³, -R²C(O)NHR⁴C(O)NHR³, -C(O)NHR²C(O)NHR³, -OCH₂R⁵C(O)NHR³, -OCH₂R⁵R²C(O)NHR³ oder -C(O)R⁶C(O)R²C(O)NHR³ ist, wobei R² und R⁴ unabhängig voneinander aus einer Gruppe ausgewählt sind, die aus C₁-C₁₀-Alkyl, C₁-C₁₀-Alkenyl und C₁-C₁₀-Alkoxy besteht, R³ unabhängig aus einer Gruppe ausgewählt ist, die aus -OH, einem Aryl, das gegebenenfalls mit mindestens einem von einem Halogen, einem Alkyl, einem Alkoxy oder -NH₂ substituiert ist, und wenn R³ ein mit -NH₂ substituiertes Aryl ist, jeder Wasserstoff in dem NH₂ gegebenenfalls mit Alkyl substituiert sein kann, R⁵ ein Aryl ist und R⁶ ein heterocyclisches Alkyl ist,
wobei,
wenn Y¹ OCH₂R⁵C(O)NHR³ ist, R³ ein Aryl ist, das gegebenenfalls mit mindestens einem von einem Halogen, einem Alkyl, einem Alkoxy oder -NH₂ substituiert ist.

2. Verbindung nach Anspruch 1, wobei die Verbindung der Formel (I-1) aus der Gruppe ausgewählt ist, die aus den in Tabelle A aufgeführten Verbindungen oder einem geometrischen Isomer, Enantiomer, Diastereomer, Racemat, pharmazeutisch verträglichen Salz oder Solvat davon besteht.
**Tabelle A:**
| (Nr.) | Struktur | (Nr.) | Struktur |
|---|---|---|---|
| I | 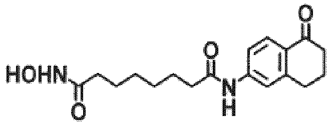 | | |
| 4 | 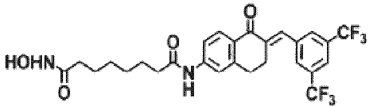 | 69 | 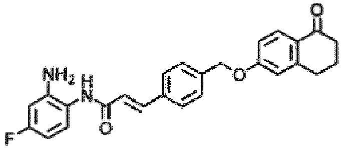 |
| 5 | 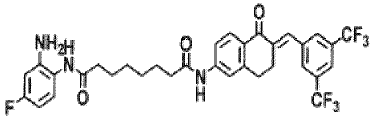 | 70 | 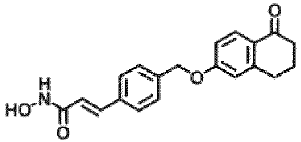 |
| 6 | 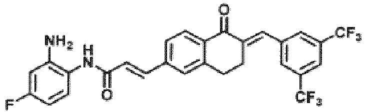 | 71 | 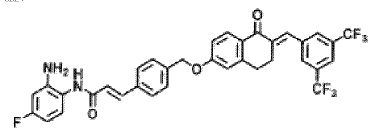 |
| 7 | 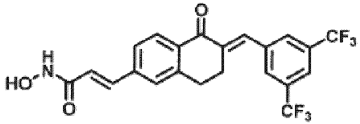 | 72 | 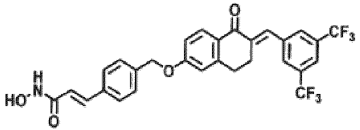 |
| 8 | 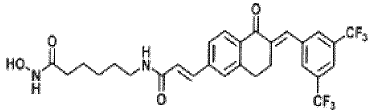 | 73 | 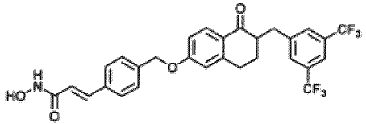 |
| 9 | 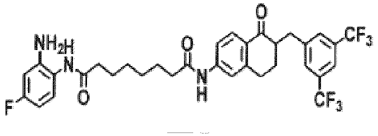 | 74 | 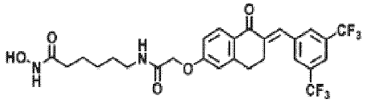 |
| 10 | 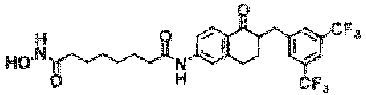 | 75 | 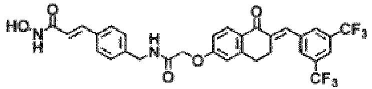 |
| 11 | 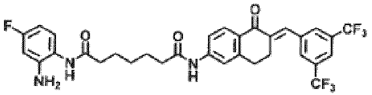 | 78 | 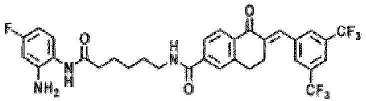 |
| 12 | 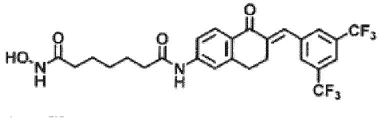 | 79 | 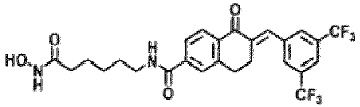 |
| 13 | 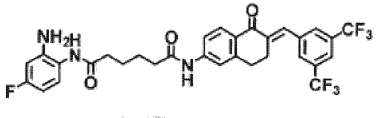 | 80 | 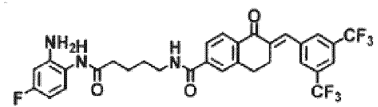 |
| 14 | 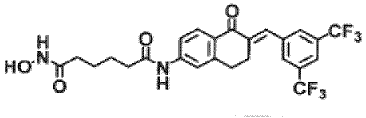 | 81 | 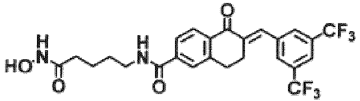 |
| 66 | 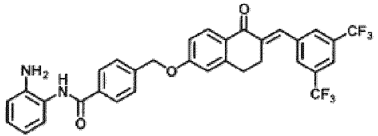 | 82 | 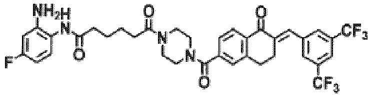 |
| 67 | 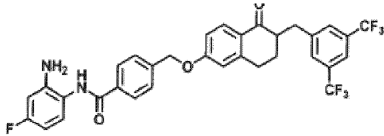 | 83 | 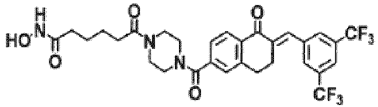 |

3. Verbindung nach Anspruch 1 oder 2, wobei die Verbindung ein dualer Autotaxin-/HDAC-Inhibitor ist.

4. Pharmazeutische Zusammensetzung, umfassend eine therapeutisch wirksame Menge der Verbindung nach Anspruch 1 oder 2 und einen pharmazeutisch verträglichen Träger.

## Revendications

1. Composé de formule (I-1) ou l'un de se isomère géométrique, énantiomère, diastéréoisomère, racémate, sel ou solvate pharmaceutiquement acceptables : 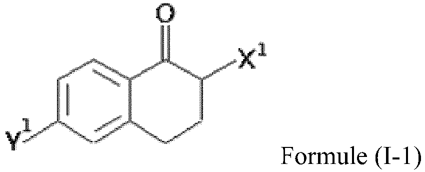 dans laquelle :
X¹ représente un atome d'hydrogène ou R¹-A¹, et R¹ représente une liaison simple ou une liaison double ; A¹ représente un groupe aryle optionnellement substitué par des groupes -CF₃ ;
Y¹ représente -NHC(O)R²C(O)NHR³, -R²C(O)NHR³, -R²C(O)NHR⁴C(O)NHR³, - C(O)NHR²C(O)NHR³, -OCH₂R⁵C(O)NHR³, -OCH₂R⁵R²C(O)NHR³, ou - C(O)R⁶C(O)R²C(O)NHR³, où R² et R⁴ sont choisis indépendamment parmi un groupe constitué d'alkyle en C₁ à C₁₀, d'alcényle en C₁ à C₁₀ et d'alcoxy en C₁ à C₁₀, R³ est choisi indépendamment parmi un groupe constitué de -OH, d'un groupe aryle optionnellement substitué par au moins un élément choisi parmi un halogène, un groupe alkyle, un groupe alcoxy ou -NH₂, et lorsque R³ est un groupe aryle substitué par -NH₂, tout atome d'hydrogène présent dans le groupe NH₂ peut être optionnellement substitué par un groupe alkyle, R⁵ est un groupe aryle et R⁶ est un groupe alkyle hétérocyclique,
dans lequel,
lorsque Y¹ est OCH₂R⁵C (O)NHR³, R³ représente un groupe aryle optionnellement substitué par au moins un atome d'halogène, un groupe alkyle, un groupe alcoxy ou -NH₂.

2. Composé selon la revendication 1, dans lequel le composé de formule (I-1) est choisi parmi le groupe constitué des composés décrits dans le tableau A ou d'un isomère géométrique, d'un énantiomère, d'un diastéréoisomère, d'un racémate, d'un sel pharmaceutiquement acceptable ou d'un solvate de ceux-ci.
**Tableau A :**
| (No.) | Structure | (No.) | Structure |
|---|---|---|---|
| 1 | 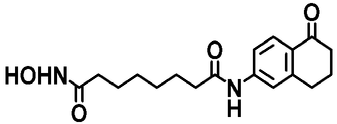 | | |
| 4 | 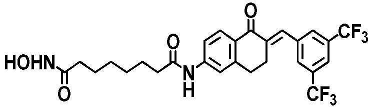 | 69 | 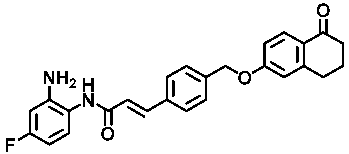 |
| 5 | 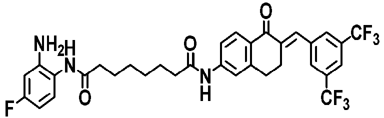 | 70 | 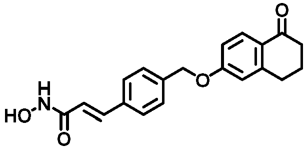 |
| 6 | 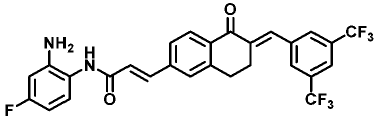 | 71 | 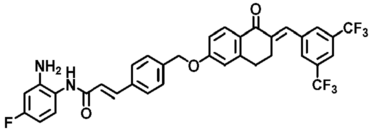 |
| 7 | 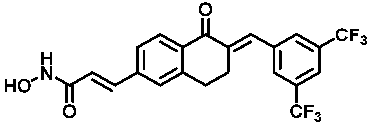 | 72 | 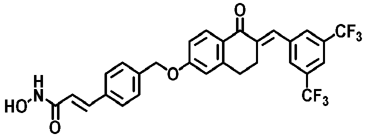 |
| 8 | 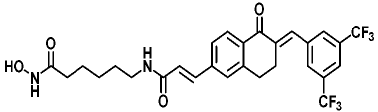 | 73 | 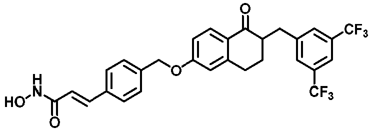 |
| 9 | 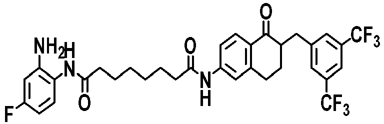 | 74 | 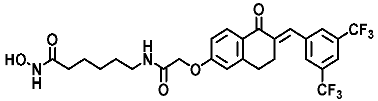 |
| 10 | 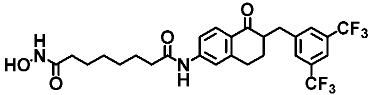 | 75 | 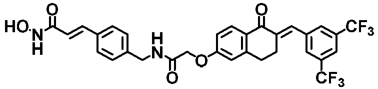 |
| 11 | 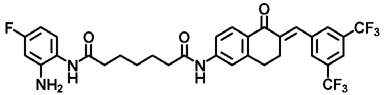 | 78 | 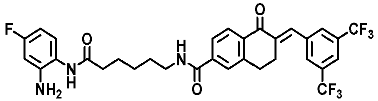 |
| 12 | 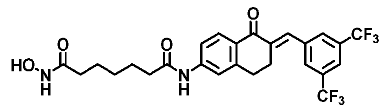 | 79 | 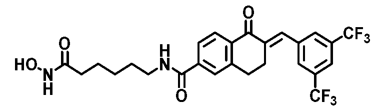 |
| 13 | 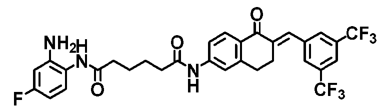 | 80 | 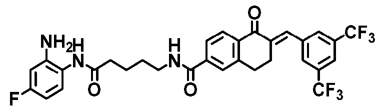 |
| 14 | 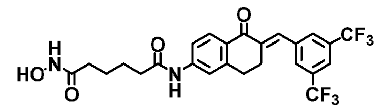 | 81 | 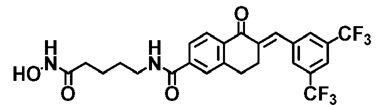 |
| 66 | 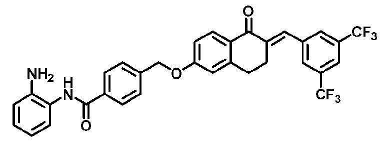 | 82 | 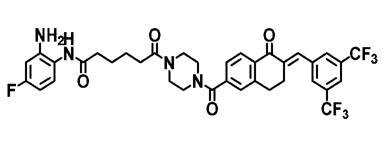 |
| 67 | 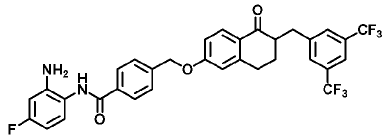 | 83 | 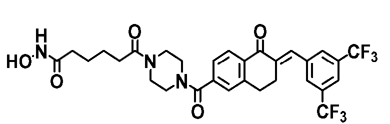 |

3. Composé selon la revendication 1 ou 2, **caractérisé en ce qu'**il s'agit d'un inhibiteur double de l'autotaxine/HDAC.

4. Composition pharmaceutique comprenant une quantité thérapeutiquement efficace du composé selon la revendication 1 ou 2 et un excipient pharmaceutiquement acceptable.
